(19) Europäisches Patentamt
European Patent Office
Office européen des brevets

(11) Numéro de publication: **0 136 945 B1**

(12) # FASCICULE DE BREVET EUROPEEN

(45) Date de publication de fascicule du brevet: **19.11.92**

(51) Int. Cl.$^5$: **C07D 211/58**, C07D 317/60, C07D 451/02, C07D 405/12, A61K 31/495, C07D 295/18, C07D 451/04, C07D 487/08, C07D 319/18, C07D 317/46, C07D 211/32

(21) Numéro de dépôt: **84401923.2**

(22) Date de dépôt: **26.09.84**

Le dossier contient des informations techniques présentées postérieurement au dépôt de la demande et ne figurant pas dans le présent fascicule.

(54) **Nouveaux composés cinnamoiques, leur procédé de préparation et leur application en thérapeutique.**

(30) Priorité: **30.09.83 FR 8315580**

(43) Date de publication de la demande:
**10.04.85 Bulletin 85/15**

(45) Mention de la délivrance du brevet:
**19.11.92 Bulletin 92/47**

(84) Etats contractants désignés:
**BE CH DE FR GB IT LI LU NL SE**

(56) Documents cités:
**EP-A- 0 083 712**
**FR-A- 2 244 518**
**FR-M- 8 198**
**US-A- 4 397 855**
**DE-A- 3 306 302**
**FR-A- 2 460 942**
**US-A- 3 753 984**

**CHEMICAL ABSTRACTS, vol. 97, 1982, page 613, no. 6322f, Columbus, Ohio, US; & JP - A - 82 32 255 (KOWA CO., LTD.) 20-02-1982**

(73) Titulaire: **DELALANDE S.A.**
**32, rue Henri Regnault**
**F-92400 Courbevoie(FR)**

(72) Inventeur: **Platel, Alain**
**23, rue Louis Pouey**
**F-92800 Puteaux(FR)**
Inventeur: **Bourgery, Guy**
**29, rue Denis Papin**
**F-92700 Colombes(FR)**
Inventeur: **Guerret, Patrick**
**113, rue du Colonel de Rochebrune**
**F-92500 Rueil Malmaison(FR)**

(74) Mandataire: **Kedinger, Jean-Paul et al**
**c/o Cabinet Malemont 42, avenue du Président Wilson**
**F-75116 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

## Description

La présente invention a pour objet de nouveaux composés cinnamoïques, leur procédé de préparation et leur application en thérapeutique.

Par FR-A-2 460 942, DE-A-3 306 302, EP-A-0 083 712, FR-A-2 244 518, FR-M-8198 et US-A-3 753 984, on connaît déjà des composés cinnamoïques.

Les composés selon l'invention comprennent plus précisément les dérivés de formule générale suivante :

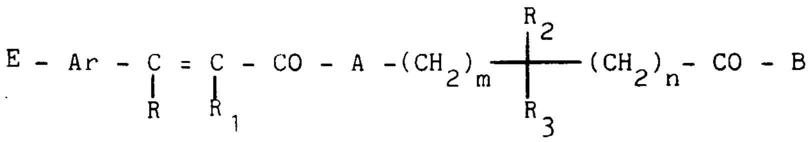

(I)

dans laquelle l'ensemble (Ar, R, $R_1$, COA, m, $R_2$, $R_3$, n, B) prend l'une quelconque des significations selon la revendication 1.

Les dérivés (I) peuvent également être sous la forme de sels d'addition d'acides ou de bases organiques ou minéraux, N-oxydes, ammonium quaternaires (notamment les iodométhylates) et hydrates des dérivés (I) ;

A/ Le procédé selon l'invention pour la préparation des dérivés (I) de formule particulière :

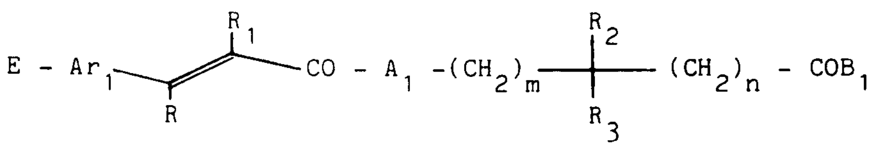

(Ia)

dans laquelle :

- $Ar_1$ a les mêmes significations que Ar dans (I), excepté les cas où Ar représente un noyauphényle substitué par deux groupes hydroxyle, ou le groupe

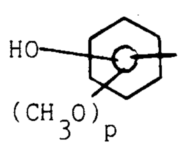

avec p = 1,

- $COA_1$ - représente l'un des ensembles suivants :

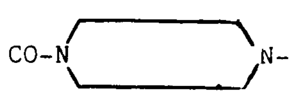

,

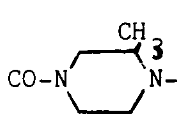

,

- $B_1$ a les mêmes significations que B dans (I), excepté les valeurs : OH, $NH(CH_2)_3COOH$, $NHCH_2COOH$ et $NH(CH_2)_2COOH$,
- R, $R_1$, $R_2$, $R_3$, m et n ont les mêmes significations que dans (I), consiste à condenser les

composés de formule :

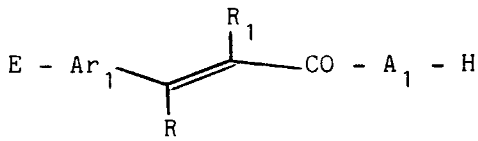

(II)

dans laquelle $Ar_1$, R, $R_1$ et $COA_1$- ont les mêmes significations que dans (Ia) avec les composés de formule :

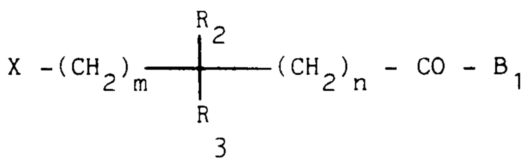

(III)

dans laquelle m, n, $R_2$, $R_3$ et $B_1$ ont les mêmes significations que dans (Ia) et X représente un groupe bon partant tel que Cl par exemple. Cette condensation est effectuée de préférence dans un solvant organique tel que l'acétone, l'acétonitrile, la méthyléthylcétone, l'éthanol, l'acétate d'éthyle, le D.M.F., le T.H.F. ou le chlorure de méthylène en présence d'une base organique ou minérale, notamment le carbonate de sodium ou de potassium.

B/ Le procédé selon l'invention pour la préparation des dérivés (I) de formule particulière :

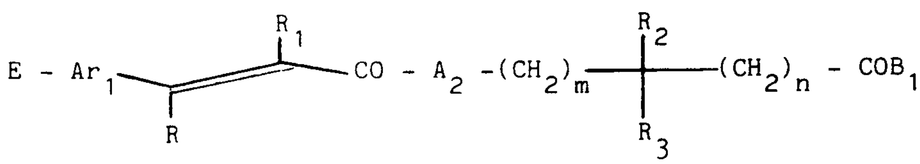

(Ib)

dans laquelle $Ar_1$, R, $R_1$, $R_2$, $R_3$, $B_1$, m et n ont les mêmes significations que dans (Ia) et $COA_2$- représente l'un des ensembles suivants :

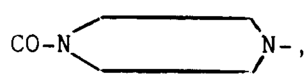

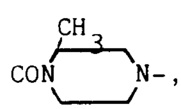

consiste :

1 - soit à condenser selon la réaction dite de "BOISSONNAS", en présence d'une base organique (de préférence la triéthylamine) et d'un chloroformiate d'alkyle tel que le chloroformiate d'éthyle ou d'isobutyle, dans un solvant aprotique (tel que chloroforme, chlorure de méthylène, DMF ou THF) les acides de formule :

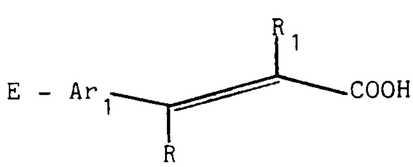

(IV)

dans laquelle $Ar_1$, R et $R_1$ ont les mêmes significations que dans (Ib) avec les dérivés de formule :

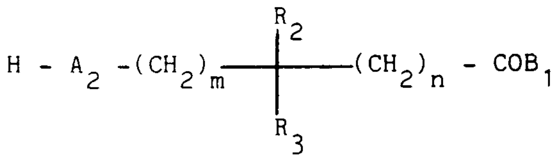

(V)

dans laquelle $A_2$, $B_1$, $R_2$, $R_3$, m et n ont les mêmes significations que dans (Ib),

2 - soit à condenser les acides (IV) avec les dérivés (V) en présence de N-hydroxybenzotriazole, de D.C.C.I. et d'une base telle que la triéthylamine, dans un solvant organique aprotique tel que le THF,

3 - soit à condenser les chlorures d'acide des acides (IV) (chlorures obtenus par exemple par action du chlorure de thionyle sur les acides (IV) selon les méthodes classiques de la littérature) avec les composés (V), en milieu aprotique tel que le toluène, ou le chlorure de méthylène, en présence d'une base telle que la triéthylamine.

D/ Le procédé selon l'invention pour la préparation des dérivés (I) trans pour lesquels B représente le groupe OH, $NHCH_2COOH$, $NH(CH_2)_2COOH$ ou $NH(CH_2)_3COO$ ou consiste à hydrolyser le groupe ester des dérivés (I) de formule particulière :

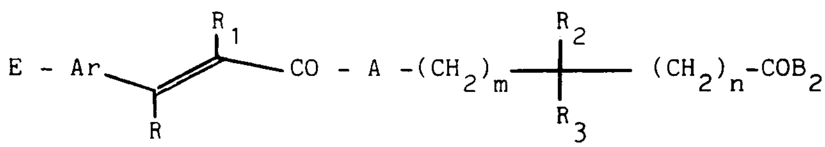

(Id)

dans laquelle Ar, R, $R_1$, $R_2$, $R_3$, A, m et n ont les mêmes significations que dans (I) et $-B_2$ représente un groupe -O+, $NHCH_2COO$+, $NH(CH_2)_2COO$+ ou $NH(CH_2)_3COO$+. Cette hydrolyse est de préférence réalisée à l'aide d'acide chlorhydrique dilué dans l'acide acétique ou à l'aide d'acide trifluoroacétique dans un solvant organique tel que le chlorure de méthylène.

E/ Le procédé selon l'invention pour la préparation des dérivés (I) trans pour lesquels Ar désigne un noyau phényle substitué par deux groupes hydroxyle ou le groupe

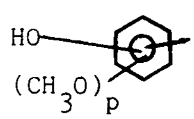

(avec p = 1), consiste à traiter par l'ammoniaque en milieu méthanolique les carbonates mixtes de formule :

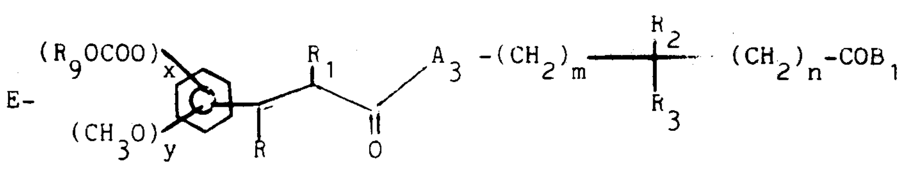

(VIII)

dans laquelle $R_9$ représente un groupe alkyle de 1 à 4 atomes de carbone, x prend la valeur 1 ou 2, y prend la valeur 0 ou 1 (avec la restriction que x ne peut prendre la valeur 2 que lorsque y = 0), R, $R_1$, $R_2$, $R_3$, m et n ont les mêmes significations que dans (I), $B_1$ a les mêmes significations que dans (Ia) et $CO-A_3$- a les mêmes significations que CO-A- dans (I).

F/ Le procédé selon l'invention pour la préparation des dérivés (L) de formule particulière :

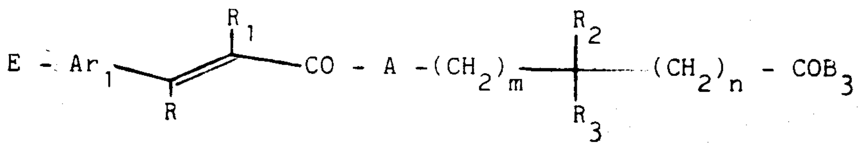 (Ie)

dans laquelle $Ar_1$, R, $R_1$, $R_2$, $R_3$, m et n ont les mêmes significations que dans (Ia), A a les mêmes significations que dans (L) et $B_3$ représente un groupe, pyrrolidino, $NHCH_2CONH_2$ ou $NH(CH_2)_2CONH_2$, consiste à condenser selon le protocole opératoire décrit au paragraphe B/ 2- les acides de formule :

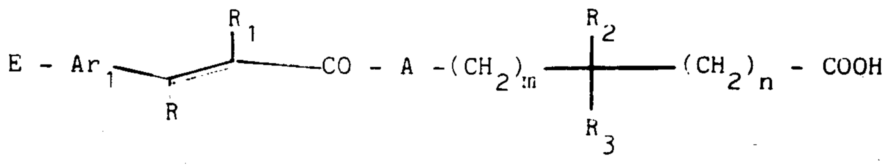 (If)

dans laquelle $Ar_1$, R, $R_1$, $R_2$, $R_3$, A, m et n ont les mêmes significations que dans (Ie), avec les amines de formule :

$H - B_3$ (XV)

dans laquelle $B_3$ a les mêmes significations que dans (Ie), les composés (If) étant quant à eux obtenus selonle protocole opératoire décrit au paragraphe D/.

G/ Le procédé selon l'invention pour la préparation des dérivés (I) pour lesquels l'enchaînement

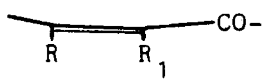

est cis (Z), consiste en une isomérisation photochimique des dérivés (I) correspondants trans(E) selon le protocole opératoire décrit dans le brevet français No. 82 03045.

H/ Les dérivés (I) objet de la présente invention peuvent être salifiés selon les méthodes usuelles. La salification peut, par exemple, être obtenue par action sur ces dérivés d'un acide minéral tel que l'acide chlorhydrique ou d'un acide organique tel que l'acide maléique, cette opération étant de préférence réalisée dans un solvant ou un mélange de solvants tels que l'acétone, l'éthanol ou l'eau, ou bien par addition d'une base organique ou minérale, dans les mêmes conditions.

I/ Les N-oxydes selon l'invention sont préparés selon les méthodes usuelles, de préférence par action des peracides organiques(tels que le M.C.P.B.A. ou l'acide para-nitroperbenzoïque) dans un solvant aprotique tel que de préférence le chlorure de méthylène, sur les dérivés (I) selon l'invention.

J/ Les ammoniums quaternaires des dérivés (I) selon l'invention et notamment les iodométhylates sont préparés par action d'iodure d'alkyle, de préférence l'iodure de méthyle, sur les dérivés (I) en solution dans un solvant organique, selon les méthodes usuelles.

K/ Les énantiomères des dérivés (I) selon l'invention sont obtenus soit par les méthodes classiques de dédoublement, à partir des sels des dérivés (I) [sels obtenus par action d'un acide organique optiquement actif sur les dérivés (I)], soit par synthèse stéréospécifique selon les protocoles opératoires décrits aux paragraphes A/, B/, C/ précédents, mais à l'aide des composés (III), (V) et (VII) optiquement actifs.

Les diastéréoisomères sont quant à eux obtenus sous forme de couple de diastéréoisomères, par chromatographie sur colonne de silice ou d'alumine.

Les composés (II) pour lesquels $COA_1$- représente le groupe

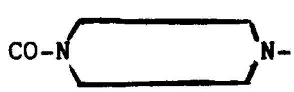

dans lequel q = 1 ou 2, sont obtenus par condensation de la pipérazine ou de l'homopipérazine avec les chlorures d'acides des acides de formule (IV).

Les composés (II) pour lesquels $COA_1$- représente le groupe

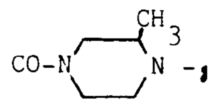

sont obtenus par hydrolyse basique des composés de formule :

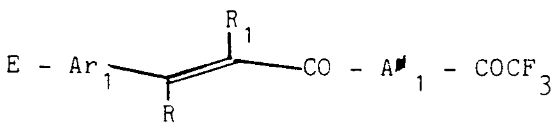

(IIa)

dans laquelle $Ar_1$, R et $R_1$ ont les mêmes significations que dans (II) et.

Les composes (IIa) sont eux mêmes obtenus par condensation, selon l'un ou l'autre des protocoles opératoires décrits au paragraphe B/ précédent des acides (IV) avec les dérivés de formule :

$H - A_1 - COCF_3$ (IX)

dans laquelle $A_1$- a les mêmes significations que dans (IIa).

Les composés (IX) sont quant à eux obtenus par hydrogénolyse catalytique (de préférence à l'aide de palladium sur charbon) des composés de formule :

$R_{10} - A_1 - COCF_3$ (X)

dans laquelle $R_{10}$ représente un groupe benzyle ou benzyloxycarbonyle et $A_1$ a les mêmes significations que dans (IIa), les composés (X) étant obtenus par action de l'anhydride trifluoracétique sur les composés de formule :

$R_{10} - A_1 - H$ (XI)

dans laquelle $R_{10}$ et $A_1$ ont les mêmes significations que dans (X).

Les composés (III) de formule particulière :

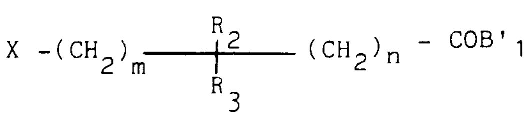

(IIIa)

dans laquelle $B'_1$ représente un groupe pyrrolidino, $NHCH_2CONH_2$, $NH(CH_2)_2CONH_2$, $O(CH_2)_2N(CH_3)_2$, $NH(CH_2)_2N(CH_3)_2$ ou $NH(CH_2)_2N(Et)_2$, où X, $R_2$, $R_3$, m et n ont les mêmes significations que dans (III) sont obtenus par condensation des composés :

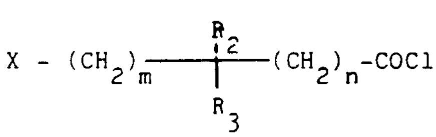

(XII)

dans laquelle X, $R_2$, $R_3$, m et n ont les mêmes significations que dans (III) avec les composés de formule :

$HB'_1$ (XIII)

dans laquelle B'$_1$ a les mêmes significations que dans (IIIa).

Ces condensations sont effectuées en présence d'une base organique telle que de préférence la triéthylamine et dans des solvants aprotiques tels que de préférence le toluène, le chlorure de méthylène ou le THF.

Les composés (XIII) pour lesquels B'$_1$ représente les groupes $NHCH_2CONH_2$ et $NH(CH_2)_2$ $CONH_2$, sont obtenus conformément aux méthodes classiques décrites dans la littérature et notamment selon les protocoles opératoires décrits dans J. Chem. Soc. 1965, 7305.

Les composés (V) pour lesquels $HA_2$- représente le groupe

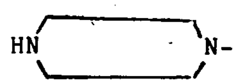

sont obtenus par condensation, de préférence dans l'éthanol à 96, des composés de formule (III) avec la pipérazine.

Les composés (V) de formule particulière :

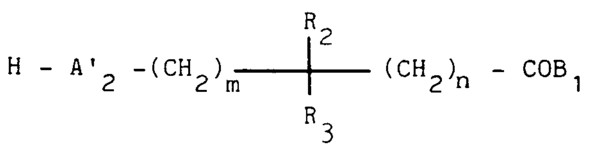

(Va)

dans laquelle H-A'$_2$- représente le groupe,

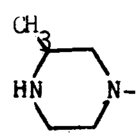

sont obtenus par hydrolyse basique des composés de formule :

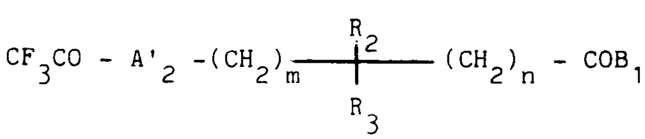

(XIV)

dans laquelle A'$_2$, $R_2$, $R_3$, $B_1$, m et n ont les mêmes significations que dans (Va).

Les composés (XIV) sont obtenus par condensation des composés de formule (III) avec les composés de formule (IX).

Enfin les composés (VIII) sont obtenus par réaction dite de "BOISSONNAS" conformément au protocole opératoire exposé au paragraphe B/1- entre les acides de formule :

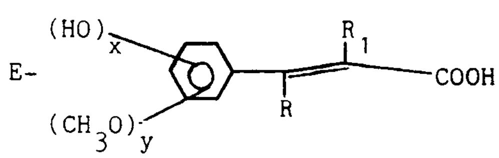

(IVa)

dans laquelle R et $R_1$, x et y ont les mêmes significations que dans (VIII), soit avec les composés (V), soit avec les composés (IX), mais en doublant les quantités de chloroformiate d'alkyle et de triéthylamine employés. Dans le cas de la condensation des composés (IVa) avec les composés (IX), la réaction est alors suivie d'un traitementbasique ($K_2CO_3$ dans le méthanol) et de la condensation, selon le protocole opératoire

décrit auparagraphe A/ précédent, des composés (III) sur les composés de formule :

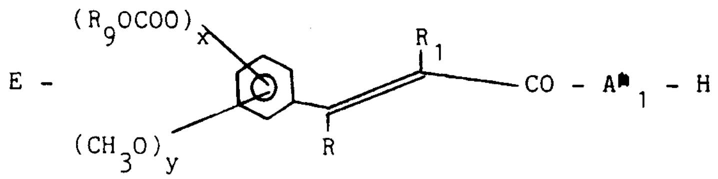

(IIb)

obtenus, formule dans laquelle $R_9$, x, y, R et $R_1$ ont les mêmes significations que dans (VIII) et $A_1$ a les mêmes significations que dans (IIa).

Les préparations suivantes sont données à titre d'exemples.

Exemple 1 : chlorhydrate du E-(3,4-dioxyméthylène cinnamoyl)-1(pyrrolidino carbonyl-2 éthyl)-4 pipérazine [(I), numéro de code 2]

On porte au reflux pendant 10 heures une suspension de 10,8 g de E-3,4-dioxyméthylène cinnamoyl pipérazine (II), de 8,7 g de chloro-1 pyrrolidinocarbonyl-2 éthyle (III), et de 5,8 g de carbonate de potassium dans 50 ml d'éthanol. Puis on filtre, évapore le filtrat, reprend le résidu dans la méthyl éthyl cétone, lave à l'eau, sèche sur sulfate de sodium (ou de magnésium), filtre, évapore le filtrat et cristallise le résidu dans l'éther isopropylique. Le produit obtenu est dissous dans l'éthanol; on ajoute de l'éthanol chlorhydrique et filtre le précipité obtenu. On obtient ainsi 8,5 g du produit attendu, dont les constantes physiques et analytiques sont données dans le tableau I ci-après.

Par le même procédé, mais à partir des réactifs correspondants, on obtient les composés de numéros de code 3, 4, 6 à 10, 17, 19, 20, 24, 29 à 31, 33, 35, 38, 40, 44 à 47, 49 à 54, 57 à 59, 63, 64, 68 et 69 figurant dans le tableau I, ainsi que les composés (V) pour lesquels H-$A_2$- représente le groupe

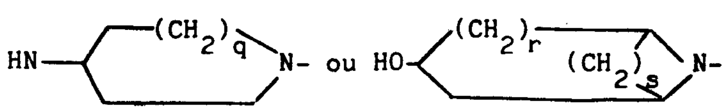

et les composés (XIV), (VII) et (VIII) (à partir des composés IIb).

Exemple 2 : E-N[[(méthoxy-4 isobutyloxycarbonyloxy-3 cinnamoyl)-1 pipérazino-4]-2 acétamido]-4 butanoate de tertiobutyle (VIII)

A une solution de 5 g d'acide E-hydroxy-3 méthoxy-4 cinnamique (IVa) dans 50 ml de THF, on ajoute 5,3 g de triéthylamine,puis on refroidit la solution à - 10° C et on ajoute 7 g de chloroformiate d'isobutyle en 20 minutes. On laisse agiter 15 minutes puis on introduit en 40 minutes une solution de 7,4 g de N-[(N-pipérazino)-2 acétamido]-4 butanoate de tertiobutyle (V). On laisse en contact 15 minutes, puis on filtre, évapore le filtrat, reprend la résidu dans l'acétate d'éthyle, lave à l'eau, puis à l'aide d'une solution aqueuse diluée de carbonate de sodium, puis à l'eau, on sèche sur sulfate de sodium ou de magnésium, filtre, évapore le filtrat et cristallise le résidu dans l'éther isopropylique. On obtient 10,9 g (rendement : 75 %) du produit attendu.

. Point de fusion : 80° C

. Formule brute : $C_{29}H_{43}N_3O_8$

. Poids moléculaire : 561,66

Par le même procédé, mais à partir des réactifs correspondants, on obtient les composés de numéros de code 2 à 4, 6, 8 à 10, 17, 19, 20, 22, 23, 29 à 31, 33, 35, 38, 40, 49 à 54, 58, 59, 61 , 65 et 66 figurant dans le table I, ainsi que les composés de formule (IIa).

Exemple 3 : maléate de la E-3,4-dioxyméthylène cinnamoyl-1 [(pyrrolidino carbonyl-4) butyl]-4 pipérazine [-(I), numéro de code 17]

On laisse agiter pendant 12 heures à 20° C unmélange de 4,5 g d'acide E-[(3,4-méthylènedioxy cinnamoyl)-4 pipérazinyl-1]-5 pentanoïque [(If) ; numéro de code 15], de 0,9 ml de pyrrolidine, de 1,8 g

d'hydroxy-1 benzotriazole, de 1,5 ml de triéthylamine et de 2,3 g de D.C.C.I. dans 100 ml de THF. Puis on filtre l'insoluble, évapore le filtrat, chromatographie le résidu sur une colonne de silice, (M.P.L.C.) ; par élution par le mélange chlorure de méthylène 98 % - méthanol 2 %, on obtient 3,8 g du produit attendu sous forme base (rendement : 85 %) que l'on dissout dans l'acétone. On ajoute une solution acétonique d'acide maléique, refroidit et filtre le précipité obtenu qui correspond au sel attendu.

Par le même procédé, mais à partir des réactifs correspondants, on obtient les composés de numéros de code 2 à 4, 8 à 10, 18 à 20, 22, 23, 26, 27, 29 à 31, 33, 35, 38, 40, 44, 45, 49 à 54, 56 à 59, 61, 65 et 66 figurant dans le tableau I, ainsi que les composés de formule (IIa).

Exemple 4A : énantiomère S(+) du E-[(3,4-méthylènedioxy cinnamoyle)-4 pipérazinyl-1]-2 pyrrolidino carbonyl-2 éthyle [(I) , numéro de code 4]

A une solution de 2,5 g de E-pipérazino-2 pyrrolidino carbonyl-2 éthyle S(-) [(V)] dans 50 ml de chlorure de méthylène, on ajoute 1,2 g de triéthylamine, puis 2,5 g du chlorure d'acide de l'acide 3,4-méthylènedioxy cinnamique (IV). On laisse agiter pendant 3 heures à 20° C, puis on lave à l'eau, décante la phase organique, l'évapore, reprend le résidu dans 50 ml d'acide chlorhydrique 1N, filtre, lave le filtrat à l'aide d'acétate d'éthyle, neutralise par de l'ammoniaque, extrait au chlorure de méthylène, sèche sur sulfate de sodium ou de magnésium, filtre, évapore le filtrat et chromatographie le résidu sur une colonne de silice (M.P.L.C.). Par élution par le mélange chlorure de méthylène 95 % - méthanol 5 %, on obtient 2,4 g (rendement : 53 %) du produit attendu.

Par le même procédé, mais à partir des réactifs correspondants, on obtient les composés de numéros de code 2, 3, 6, 8 à 10, 17, 19, 20, 22, 23, 29 à 31, 33, 35, 38, 40, 49 à 54, 58, 59, 61 , 65 et 66 figurant dans le tableau I, ainsi que les composés de formule (IIa) et (IIIa).

Exemple 4B : E-N-(3,4-dioxyméthylène cinnamoyl) pipérazine (II)

On chauffe au reflux pendant 40 minutes unmélange de 250 g d'acide E-3,4-dioxyméthylène cinnamique (IV) dans 875 ml de chlorure de thionyle. Puis on distille le chlorure de thionyle n'ayant pas réagi, reprend le résidu dans le toluène, évapore le toluène, cristallise le résidu dans l'éther de pétrole et filtre (273 g). Le produit obtenu est ajouté à 20° C, lentement à une solution de 224 g de pipérazine anhydre dans 1800 ml d'acide acétique (solution préalablement obtenue par addition lente de la pipérazine dans l'acide acétique à 40° C). Puis on laisse agiter 12 heures, évapore l'acide acétique sous bon vide, reprend le résidu dans l'eau glacée, filtre, basifie le filtrat à l'aide de soude en pastilles, extrait le précipité formé à l'aide de méthyléthyl cétone, sèche sur sulfate de sodium (ou de magnésium), filtre, évapore le filtrat et cristallise le résidu dans le toluène. On obtient 110 g du produit attendu.

. Point de fusion : 135° C
. Rendement : 32 %
. Formule brute : $C_{14}H_6N_2O_3$
. Poids moléculaire : 260,28
. Analyse élémentaire :

| | C | H | N |
|---|---|---|---|
| Calculé (%) | 64,60 | 6,20 | 10,76 |
| Trouvé (%) | 64,29 | 6,27 | 10,50 |

Exemple 5 : chlorhydrate hydraté du E-(3,4-dioxyméthylène cinnamoyl)-4 pyrrolidinocarbonylméthyl-1 pipéridine [(I), numéro de code 18]

On laisse agiter pendant 3 jours à température ambiante, un mélange de 5,4 g de pipéronal (VI), de 6,8 g d'acétyl-4 pyrrolidinocarbonylméthyl-1 pipéridine (VII), de 7,1 ml de soude concentrée dans 80 ml d'éthanol, puis on évapore les solvants, reprend le résidu dans l'acétate d'éthyle, lave à l'eau, sèche sur sulfate de sodium ou de magnésium, filtre, évapore le filtrat et chromatographie le résidu sur une colonne de silice (M.P.L.C.). Par élution par le mélange chlorure de méthylène 96 % - méthanol 4 %, on obtient 5,5 g d'un produit huileux que l'on dissout dans de l'acétone, on ajoute de l'éthanol chlorhydrique ≈6N, refroidit, filtre le précipité formé et le recristallise dans l'éthanol absolu ; on obtient ainsi 3,5 g (rendement : 30 %) du produit attendu.

Par le même procédé, mais à partir des réactifs correspondants, on obtient les composés de numéros de code 26 et 27 figurant dans le tableau I.

Exemple 6 : acide E-N-[[3,4-dioxyméthylène cinnamoyl)-4 pipérazinyl]-1 méthylcarbonylamino]-4 butyrique [(I), numéro de code 1]

A 50 ml d'acide trifluoroacétique refroidis à 5° C, on ajoute en agitant une solution de 9,2 g d'ester tertiobutylique de l'acide E-[[3,4-dioxyméthylène cinnamoyl)-4 pipérazinyl]-1 méthylcarbonylamino]-4 butyrique (Id), préparé conformément aux exemples 1, 2, 3 ou 4A précédents, dans 20 ml de chlorure de méthylène, sans que la température du milieu réactionnel ne dépasse 20° C (addition en 15 minutes environ). Puis on laisse agiter 12 heures, évapore les solvants, reprend le résidu dans l'eau, lave la phase aqueuse a l'aide d'éther éthylique, porte le pH du milieu à ~5 à l'aide d'ammoniaque, extrait a l'acétate d'éthyle, sèche sur sulfate de magnésium, filtre et évapore le filtrat. Le résidu est cristallisé dans l'acétate d'éthyle, puis recristallisé dans le méthanol. On obtient ainsi 2,9 g du produit attendu.

Par le même procédé, mais à partir des réactifs correspondants, on obtient les composés de numéros de code 11 à 16, 28, 32, 34, 36, 37, 39, 41 à 43, 48 et 67 figurant dans le tableau I.

Exemple 7 : E-N-[[(méthoxy-4 hydroxy-3 cinnamoyl)-1 pipérazino-4]-2 acétamido]-4 butanoate de tertiobutyle (I)

On laisse une solution de 10,8 g de E-N-[[(méthoxy-4 isobutyloxycarbonyloxy-3 cinnamoyl)-1 pipérazino-4]-2 acétamido]-4 butanoate de tertiobutyle [(VIII), préparé à l'exemple 2] dans 150 ml de méthanol saturé d'ammoniac gazeux, pendant 2 jours à 20° C. Puis on évapore les solvants et chromatographie le résidu sur une colonne de silice (H.P.L.C.). Par élution par les mélanges : chlorure de méthylène 95 % - méthanol 5 %, puis chlorure de méthylène 92,5 % - méthanol 7,5 %, on obtient 8,1 g (rendement : 91 %) du produit attendu.

. Formule brute : $C_{24}H_{35}H_{3}O_{6}$
. Poids moléculaire : 461,54

Par le même procédé, mais à partir des réactifs correspondants, on obtient les composés de numéros de code 21 et 25 figurant dans le tableau I.

Exemple 8 : E-amino-4 (3,4-dioxyméthylène cinnamoyl)-1 pipéridine (II)

On laisse agiter 12 heures à température ambiante unmélange de 10,2 g de E-trifluorométhylcarbonylamino-4 (3,4-dioxyméthylène cinnamoyl)-1 pipéridine (IIa) et de 24,5 g de $K_2CO_3$ dans 250 ml de méthanol et 100 ml d'eau. Puis on évapore les solvants, reprend le résidu dans du chloroforme, lave à l'eau, sèche sur sulfate de sodium ou de magnésium, filtre et évapore le filtrat. On obtient ainsi le produit attendu cristallisé.

. Point de fusion : 120° C
. Rendement : ~100 %
. Formule brute : $C_{15}H_{18}N_{2}O_{3}$
. Poids moléculaire : 274,31

Par le même procédé, mais à partir des réactifs correspondants, on obtient les autres composés de formule (II) à partir des composés (IIa) correspondants, ainsi que les composés de formule (V) à partir des composés (XIV) et les composés de formule (IIb) issus de la condensation des composés (IVa) et (IX).

Exemple 9 : benzyl-1 trifluorométhylcarbonylamino-4 pipéridine (X)

A une solution refroidie à 0° C de 86 g de benzyl-1 amino-4 pipéridine (XI) dans 350 ml de pyridine, on ajoute lentement (en 2 heures) 75 ml d'anhydride trifluoroacétique. Puis on laisse 30 minutes entre 0 et 10° C, verse la solution dans 1500 ml d'eau glacée, extrait à l'éther, lave à l'eau, sèche sur sulfate de sodium ou de magnésium, filtre, évapore le filtrat, reprend le résidu dans de l'éther isopropylique, filtre l'insoluble et évapore le filtrat. On obtient ainsi le produit attendu cristallisé.

. Point de fusion : 125° C
. Rendement : 72 %
. Formule brute : $C_{14}H_{17}F_{3}N_{2}O$
. Poids moléculaire : 286,29

Par le même procédé, mais à partir des réactifs correspondants, on obtient les autres composés (X).

Exemple 10 : trifluorométhylcarbonylamino-4 pipéridine (IX)

On laisse agiter pendant 8 jours sous atmosphère d'hydrogène une suspension de 92 g de benzyl-1 trifluorométhylcarbonylamino-4 pipéridine (X) et de 9 g de palladium sur charbon à 10 % humide dans 1000 ml d'éthanol, à température ambiante. Puis on filtre, évapore le filtrat et chromatographie le résidu sur une colonne de silice (M.P.L.C.). Par élution par le méthanol pur, on obtient 44 g du produit attendu.

. Point de fusion : 111° C
. Rendement : 70 %
. Formule brute : $C_7H_{11}F_3N_2O$
. Poids moléculaire : 196,17

Par le même procédé, mais à partir des réactifs correspondants, on obtient les autres composés (IX).

Exemple 11 : iodométhylate du E-(3,4-méthylènedioxycinnamoyloxy)-4 pyrrolidinocarbonylméthyl-1 pipéridine [(L), numéro de code 62]

A une solution de 4,3 g de E-(3,4-méthylènedioxycinnamoyloxy)-4 pyrrolidinocarbonylméthyl-1 pipéridine [(I), numéro de code 61] dans 50 ml de chlorure de méthylène, on ajoute, à température ambiante, 2,5 ml d'iodure de méthyle, puis on laisse agiter pendant 12 heures, à l'abri de l'air. Puis on filtre, lave le précipité sur le filtre àl'aide de chlorure de méthylène, puis le sèche sous bon vide. On obtient 5 g (rendement : 85 %) du produit attendu.

Par le même procédé, mais à partir des réactifs correspondants, on obtient le composé de numéro de code 55 figurant dans le tableau I.

Exemple 12 : N-oxyde du E-(3,4-méthylènedioxycinnamoyl)-4 pyrrolidinocarbonylmethyl-1 pipérazine [(I), numéro de code 60]

A une solution de 7,4 g de E-(3,4-méthylènedioxycinnamoyl)-4 pyrrolidinocarbonylméthyl-1 pipérazine dans 400 ml de chloroforme, on ajoute par petites portions, en 20 minutes et à température ambiante, 4,9 g d'acide paranitroperbenzoïque. Puis onlaisse agiter pendant 30 minutes, filtre, lave le filtrat à l'aide d'une solution de bicarbonate de soude, puis à l'eau, sèche sur sulfate de sodium ou de magnésium, filtre et évapore le filtrat. Le résidu est repris dans l'eau et la phase aqueuse est alors extraite en continu par du chlorure de méthylène. La phase organique est alors séchée sur sulfate de sodium. Puis on filtre, évapore le filtrat et cristallise le résidu dans l'éther éthylique. On obtient 5 g (rendement : 64,5 %) du produit attendu.

Exemple 13 : E-R(-)[[(3,4-dioxyméthylène cinnamoyl)-4 pipérazinyl]-1]-1 pyrrolidinocarbonyl-1 éthyle [(I), numéro de code 5]

On porte à 50° C une suspension de 7 g d'acide (+) binaphtyl phosphorique dans 50 ml d'éthanol. Puis on y introduit une solution de 7,7 g de E-[[(3,4-dioxyméthylène cinnamoyl)-4 pipérazinyl]-1]-1 pyrrolidinocarbonyl-1 éthyle [(I), numéro de code 3] dans 20 ml d'éthanol. On laisse agiter ensuite 4 heures, puis on filtre, rince le précipité à l'éthanol et le sèche sous bon vide à 80° C. Le précipité est recristallisé dans l'éthanol. On obtient 6,34 g de sel, qui est repris dans de l'eau, on basifie à l'aide d'ammoniaque, extrait à l'acétate d'éthyle, évapore la phase organique et chromatographie le résidu sur une colonne de silice. Elués par les mélanges chlorure de méthyle 95 % - méthanol 5 %, puis chlorure de méthylène 90 % - méthanol 10 %, on obtient 1,76 g (rendement : 26 %) du produit attendu.

TABLEAU I

$$E - Ar - C(R) = C(R_1) - CO - A - (CH_2)_m - C(R_2)(R_3) - (CH_2)_n - CO - B \qquad (I)$$

| No. de Code | Ar- | R | $R_1$ | COA- | m | $R_2$ | $R_3$ | n | -B | Forme | Formule brute | Poids moléculaire | Point de fusion (°C) | ANALYSE ELEMENTAIRE OU $[\alpha]_D$ % | C | H | N |
|---|---|---|---|---|---|---|---|---|---|---|---|---|---|---|---|---|---|
| 1 | 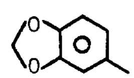 | H | H | 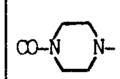 | 0 | H | H | 0 | $-NH(CH_2)_3$ HOOC | Base | $C_{20}H_{25}N_3O_6$ | 403,42 | 165 | C.H.N. | | | |
| | | | | | | | | | | | | | | Cal. | 59,54 | 6,25 | 10,42 |
| | | | | | | | | | | | | | | Tr. | 59,18 | 6,35 | 10,24 |
| 2 | " | " | " | " | 1 | " | " | " | 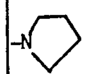 | HCl | $C_{21}H_{28}ClN_3O_4$ | 421,91 | > 260 | C.H.N. | | | |
| | | | | | | | | | | | | | | Cal. | 59,78 | 6,69 | 9,96 |
| | | | | | | | | | | | | | | Tr. | 59,75 | 6,94 | 9,83 |
| 3 | " | " | " | " | 0 | $CH_3$ | " | " | " (±) | HCl + 1,15% $H_2O$ | $C_{21}H_{28}ClN_3O_4$ + 1,15 % $H_2O$ | 426,82 | 252 | C.H.N. (+ 1,15 % $H_2O$) | | | |
| | | | | | | | | | | | | | | Cal. | 59,09 | 6,74 | 9,85 |
| | | | | | | | | | | | | | | Tr. | 59,11 | 6,68 | 9,75 |
| 4 | " | " | " | " | " | " | " | " | 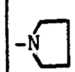 (S+) | Base + 2,5% $H_2O$ | $C_{21}H_{27}N_3O_4$ + 2,5 % $H_2O$ | 395,33 | Produit vitreux | C.H.N. (+ 2,5 % $H_2O$) | | | |
| | | | | | | | | | | | | | | Cal. | 63,79 | 7,25 | 10,63 |
| | | | | | | | | | | | | | | Tr. | 63,52 | 7,55 | 10,50 |
| | | | | | | | | | | | | | | $[\alpha]_D^{20}$ = + 29,8° (C=1 % $CHCl_3$) | | | |

TABLEAU I (suite)

| No. de Code | Ar- | R | R1 | COA- | m | $R_2$ | $R_3$ | n | -B | Forme | Formule brute | Poids moléculaire | Point de fusion (°C) | ANALYSE ELEMENTAIRE OU $[\alpha]_D$ % | C | H | N |
|---|---|---|---|---|---|---|---|---|---|---|---|---|---|---|---|---|---|
| 5 | 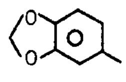 | H | H | 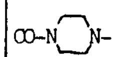 | O | $CH_3$ | H | O | 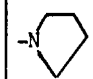 (R-) | Base + 3,13% $H_2O$ | $C_{21}H_{27}N_3O_4$ + 3,13 % $H_2O$ | 397,90 | Produit vitreux | C.H.N. (+3,13 % $H_2O$) | | | |
| | | | | | | | | | | | | | | Cal. | 63,38 | 7,19 | 10,56 |
| | | | | | | | | | | | | | | Tr. | 63,41 | 7,31 | 10,17 |
| | | | | | | | | | | | | | | $[\alpha]_D^{20}$ = - 29,7° (C = 1 % $CHCl_3$) | | | |
| 6 | " | " | " | " | " | H | " | " | 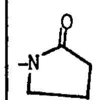 | Base | $C_{20}H_{23}N_3O_5$ | 385,41 | 163 | C.H.N. | | | |
| | | | | | | | | | | | | | | Cal. | 62,32 | 6,02 | 10,90 |
| | | | | | | | | | | | | | | Tr. | 62,03 | 6,02 | 10,82 |
| 7 | 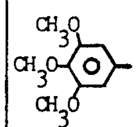 | " | " | 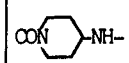 | " | " | " | " | 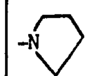 | HCl + 4,2% $H_2O$ | $C_{23}H_{34}ClN_3O_5$ + 4,2 % $H_2O$ | 488,55 | 200 | C.H.N. (+ 4,2 % $H_2O$) | | | |
| | | | | | | | | | | | | | | Cal. | 56,54 | 7,41 | 8,60 |
| | | | | | | | | | | | | | | Tr. | 56,58 | 7,20 | 8,57 |
| 8 | 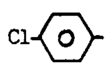 | " | " | 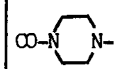 | " | $CH_3$ | " | " | " (±) | Base | $C_{20}H_{26}ClN_3O_2$ | 375,89 | 170 | C.H.N. | | | |
| | | | | | | | | | | | | | | Cal. | 63,90 | 6,97 | 11,18 |
| | | | | | | | | | | | | | | Tr. | 63,85 | 7,09 | 10,91 |

TABLEAU I (suite)

| No. de Code | Ar- | R | $R_1$ | COA- | m | $R_2$ | $R_3$ | n | -B | Forme | Formule brute | Poids moléculaire | Point de fusion (°C) | ANALYSE ELEMENTAIRE OU $[\alpha]_D$ % | C | H | N |
|---|---|---|---|---|---|---|---|---|---|---|---|---|---|---|---|---|---|
| 9 | 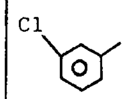 | H | H | 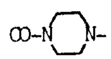 | 0 | $CH_3$ | H | 0 | 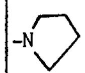 (±) | Base | $C_{20}H_{26}ClN_3O_2$ | 375,89 | 100 | C.H.N. | | | |
| | | | | | | | | | | | | | | Cal. | 63,90 | 6,97 | 11,18 |
| | | | | | | | | | | | | | | Tr. | 63,90 | 7,32 | 10,87 |
| 10 | 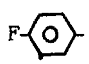 | " | " | " | " | " | " | " | " | " | $C_{20}H_{26}FN_3O_2$ | 359,43 | 143 | C.H.N. | | | |
| | | | | | | | | | | | | | | Cal. | 66,83 | 7,29 | 11,69 |
| | | | | | | | | | | | | | | Tr. | 66,90 | 7,60 | 11,46 |
| 11 | 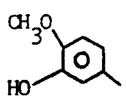 | " | " | " | " | H | " | " | $-NH-(CH_2)_3$ HOOC | HCl + 4,5% $H_2O$ | $C_{20}H_{28}ClN_3O_6$ + 4,5 % $H_2O$ | 462,53 | 110 | C.H.N. (+ 4,5 % $H_2O$) | | | |
| | | | | | | | | | | | | | | Cal. | 51,94 | 6,61 | 9,09 |
| | | | | | | | | | | | | | | Tr. | 51,91 | 6,74 | 8,94 |
| 12 | 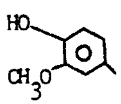 | " | " | " | " | " | " | " | " | HCl + 1,9% $H_2O$ | $C_{20}H_{28}ClN_3O_6$ + 1,9 % $H_2O$ | 450,51 | 200 | C.H.N. (+ 1,9 % $H_2O$) | | | |
| | | | | | | | | | | | | | | Cal. | 53,32 | 6,48 | 9,33 |
| | | | | | | | | | | | | | | Tr. | 53,45 | 6,23 | 9,61 |

TABLEAU I (suite)

| No. de Code | Ar- | R | $R_1$ | COA- | m | $R_2$ | $R_3$ | n | -B | Forme | Formule brute | Poids moléculaire | Point de fusion (°C) | ANALYSE ELEMENTAIRE OU $[\alpha]_D$ % | C | H | N |
|---|---|---|---|---|---|---|---|---|---|---|---|---|---|---|---|---|---|
| 13 | 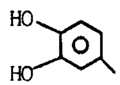 | H | H | 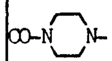 | 0 | H | H | 0 | 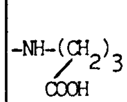 | HCl + 4 % $H_2O$ | $C_{19}H_{26}ClN_3O_6$ + 4 % $H_2O$ | 445,71 | > 210 | C.H.N. (+ 4 % $H_2O$) | | | |
| | | | | | | | | | | | | | | Cal. | 51,20 | 6,33 | 9,43 |
| | | | | | | | | | | | | | | Tr. | 51,49 | 6,15 | 9,30 |
| 14 | 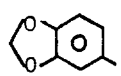 | " | " | " | 1 | " | " | 1 | -OH | HCl + 5,23% $H_2O$ | $C_{18}H_{23}ClN_2O_5$ + 5,23 % $H_2O$ | 403,96 | 140 | C.H.N. (+ 5,23 % $H_2O$) | | | |
| | | | | | | | | | | | | | | Cal. | 53,52 | 6,33 | 6,94 |
| | | | | | | | | | | | | | | Tr. | 53,63 | 6,08 | 7,00 |
| 15 | " | " | " | " | " | " | " | 2 | " | HCl + + 4,8% $H_2O$ | $C_{19}H_{25}ClN_2O_5$ + 4,8 % $H_2O$ | 416,70 | 214 | C.H.N. (+ 4,8 % $H_2O$) | | | |
| | | | | | | | | | | | | | | Cal. | 54,76 | 6,58 | 6,72 |
| | | | | | | | | | | | | | | Tr. | 55,00 | 6,74 | 6,77 |
| 16 | " | " | " | " | 1 | " | " | 0 | " | HCl + 0,6% $H_2O$ | $C_{17}H_{21}ClN_2O_5$ + 0,6 % $H_2O$ | 370,89 | > 260 | C.H.N. (+ 0,6 % $H_2O$) | | | |
| | | | | | | | | | | | | | | Cal. | 55,05 | 5,77 | 7,55 |
| | | | | | | | | | | | | | | Tr. | 55,01 | 5,57 | 7,76 |

TABLEAU I (suite)

| No. de Code | Ar- | R | $R_1$ | COA- | m | $R_2$ | $R_3$ | n | -B | Forme | Formule brute | Poids molécu-laire | Point de fusion (°C) | ANALYSE ELEMENTAIRE OU $[\alpha]_D$ % | C | H | N |
|---|---|---|---|---|---|---|---|---|---|---|---|---|---|---|---|---|---|
| 17 | 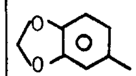 | H | H | 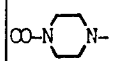 | 1 | H | H | 2 | 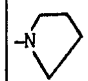 | Maléate | $C_{27}H_{35}N_3O_8$ | 529,57 | 179 | C.H.N. | | | |
| | | | | | | | | | | | | | | Cal. | 61,23 | 6,66 | 7,94 |
| | | | | | | | | | | | | | | Tr. | 61,35 | 6,88 | 7,84 |
| 18 | " | " | " | 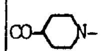 | 0 | " | " | 0 | " | HCl + 2 % $H_2O$ | $C_{21}H_{27}ClN_2O_4$ + 2 % $H_2O$ | 415,20 | 205 | C.H.N. (+ 2 % $H_2O$) | | | |
| | | | | | | | | | | | | | | Cal. | 60,74 | 6,78 | 6,75 |
| | | | | | | | | | | | | | | Tr. | 60,50 | 7,16 | 6,60 |
| 19 | 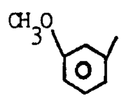 | " | " | 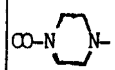 | " | $CH_3$ | " | " | " (±) | HCl | $C_{21}H_{30}ClN_3O_3$ | 407,93 | > 200 | C.H.N. | | | |
| | | | | | | | | | | | | | | Cal. | 61,83 | 7,41 | 10,30 |
| | | | | | | | | | | | | | | Tr. | 61,71 | 7,64 | 10,35 |
| 20 | 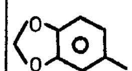 | " | " | " | 1 | H | H | 1 | 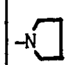 | Maléate | $C_{26}H_{33}N_3O_8$ | 515,55 | 158 | C.H.N. | | | |
| | | | | | | | | | | | | | | Cal. | 60,57 | 6,45 | 8,15 |
| | | | | | | | | | | | | | | Tr. | 60,57 | 6,65 | 8,07 |

TABLEAU I (suite)

| No. de Code | Ar- | R | $R_1$ | COA- | m | $R_2$ | $R_3$ | n | -B | Forme | Formule brute | Poids moléculaire | Point de fusion (°C) | ANALYSE ELEMENTAIRE OU [α]D % | C | H | N |
|---|---|---|---|---|---|---|---|---|---|---|---|---|---|---|---|---|---|
| 21 | 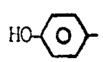 | H | H | 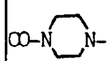 | 0 | $CH_3$ | H | 0 | 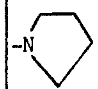 | HCl | $C_{20}H_{28}ClN_3O_3$ | 393,90 | 120 (décomposition) | C.H.N. | | | |
| | | | | | | | | | | | | | | Cal. | 60,98 | 7,17 | 10,67 |
| | | | | | | | | | | | | | | Tr. | 60,72 | 7,50 | 10,35 |
| 22 | 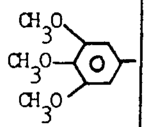 | " | " | 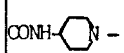 | " | H | " | " | " | " | $C_{23}H_{34}ClN_3O_5$ | 467,98 | ≃ 260 | C.H.N. | | | |
| | | | | | | | | | | | | | | Cal. | 59,03 | 7,32 | 8,98 |
| | | | | | | | | | | | | | | Tr. | 58,70 | 7,68 | 8,67 |
| 23 | 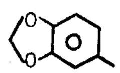 | " | " | " | " | " | " | " | " | HCl + 1,25% $H_2O$ | $C_{21}H_{28}ClN_3O_4$ + 1,25 % $H_2O$ | 427,26 | ≃ 260 | C.H.N. (+ 1,25 % $H_2O$) | | | |
| | | | | | | | | | | | | | | Cal. | 59,03 | 6,75 | 9,84 |
| | | | | | | | | | | | | | | Tr. | 58,61 | 6,91 | 9,60 |
| 24 | " | " | " | 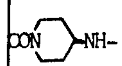 | " | " | " | " | " | HCl | $C_{21}H_{28}ClN_3O_4$ | 421,91 | > 260 | C.H.N. | | | |
| | | | | | | | | | | | | | | Cal. | 59,78 | 6,69 | 9,96 |
| | | | | | | | | | | | | | | Tr. | 59,53 | 6,72 | 9,75 |

TABLEAU I (suite)

| No. de Code | Ar- | R | $R_1$ | COA- | m | $R_2$ | $R_3$ | n | -B | Forme | Formule brute | Poids moléculaire | Point de fusion (°C) | ANALYSE ELEMENTAIRE OU $[\alpha]_D$ % | C | H | N |
|---|---|---|---|---|---|---|---|---|---|---|---|---|---|---|---|---|---|
| 25 | 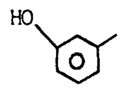 | H | H | 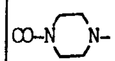 | 0 | $CH_3$ | H | 0 | 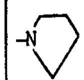 | HCl | $C_{20}H_{28}ClN_3O_3$ | 393,90 | 100 (décomposition) | C.H.N. | | | |
| | | | | | | | | | | | | | | Cal. | 60,98 | 7,17 | 10,67 |
| | | | | | | | | | | | | | | Tr. | 60,92 | 7,30 | 10,40 |
| 26 | 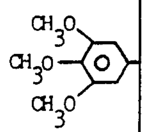 | " | " | 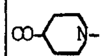 | " | H | " | " | " | HCl + 3,5% $H_2O$ | $C_{23}H_{33}ClN_2O_5$ + 3,5 % $H_2O$ | 469,40 | 148 | C.H.N. (+ 3,5 % $H_2O$) | | | |
| | | | | | | | | | | | | | | Cal. | 58,85 | 7,47 | 5,97 |
| | | | | | | | | | | | | | | Tr. | 58,82 | 7,39 | 6,12 |
| 27 | " | " | " | " | " | " | " | " | 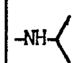 | Oxalate + 1% $H_2O$ | $C_{24}H_{34}N_2O_9$ + 1 % $H_2O$ | 499,52 | 105 | C.H.N. (+ 1 % $H_2O$) | | | |
| | | | | | | | | | | | | | | Cal. | 57,71 | 6,97 | 5,61 |
| | | | | | | | | | | | | | | Tr. | 57,31 | 6,82 | 5,60 |
| 28 | 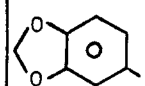 | " | " | 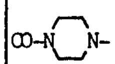 | " | $CH_3$ | " | " | $-NH-CH_2COOH$ | hémifumarate + 0,54% $H_2O$ | $C_{21}H_{25}N_3O_8$ + 0,54 % $H_2O$ | 449,86 | 110 | C.H.N. (+ 0,54 % $H_2O$) | | | |
| | | | | | | | | | | | | | | Cal. | 56,07 | 5,66 | 9,34 |
| | | | | | | | | | | | | | | Tr. | 55,84 | 5,86 | 9,18 |

TABLEAU I (suite)

| No. de Code | Ar- | R | $R_1$ | COA- | m | $R_2$ | $R_3$ | n | -B | Forme | Formule brute | Poids moléculaire | Point de fusion (°C) | ANALYSE ELEMENTAIRE OU $[\alpha]_D$ % | C | H | N |
|---|---|---|---|---|---|---|---|---|---|---|---|---|---|---|---|---|---|
| 29 | 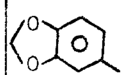 | H | H | 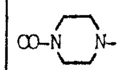 | O | $CH_3$ | H | O | $-NHCH_2CONH_2$ | HCl + 2,1% $H_2O$ | $C_{19}H_{25}ClN_4O_5$ + 2,1 % $H_2O$ | 433,99 | 150 | C.H.N. (+ 2,1 % $H_2O$) | | | |
| | | | | | | | | | | | | | | Cal. | 52,58 | 6,03 | 12,91 |
| | | | | | | | | | | | | | | Tr. | 52,85 | 6,13 | 12,87 |
| 30 | " | " | $CH_3$ | " | " | H | " | " | 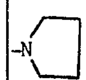 | Base<br>HCl | $C_{21}H_{27}N_3O_4$<br>$C_{21}H_{28}ClN_3O_4$ | 385,40<br>421,91 | 102<br>200 | C.H.N. (HCl) | | | |
| | | | | | | | | | | | | | | Cal. | 59,78 | 6,69 | 9,96 |
| | | | | | | | | | | | | | | Tr. | 59,64 | 6,98 | 9,60 |
| 31 | " | $CH_3$ | H | 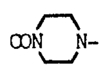 | O | " | " | " | 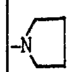 | HCl + 4,22% $H_2O$ | $C_{21}H_{28}ClN_3O_4$ + 4,22 % $H_2O$ | 440,50 | 176 | C.H.N. (+ 4,22 % $H_2O$) | | | |
| | | | | | | | | | | | | | | Cal. | 57,26 | 6,88 | 9,54 |
| | | | | | | | | | | | | | | Tr. | 57,38 | 6,81 | 9,33 |
| 32 | 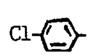 | H | " | " | " | $CH_3$ | " | " | -OH (±) | Base + 2,7% $H_2O$ | $C_{16}H_{19}ClN_2O_3$ + 2,7% $H_2O$ | 331,74 | 120 (décomposition) | C.H.N. (+ 2,7 % $H_2O$) | | | |
| | | | | | | | | | | | | | | Cal. | 57,92 | 6,07 | 8,44 |
| | | | | | | | | | | | | | | Tr. | 57,63 | 6,17 | 7,96 |

TABLEAU I (suite)

| No. de Code | Ar- | R | $R_1$ | COA- | m | $R_2$ | $R_3$ | n | -B | Forme | Formule brute | Poids moléculaire | Point de fusion (°C) | ANALYSE ELEMENTAIRE OU $[\alpha]_D$ % | C | H | N |
|---|---|---|---|---|---|---|---|---|---|---|---|---|---|---|---|---|---|
| 33 | 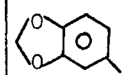 | -H | -H | 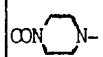 | 0 | Et | H | 0 | 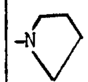 (±) | Base | $C_{22}H_{29}N_3O_4$ | 399,48 | 95 | C.H.N. | | | |
| | | | | | | | | | | | | | | Cal. | 66,14 | 7,32 | 10,52 |
| | | | | | | | | | | | | | | Tr. | 66,11 | 7,33 | 10,44 |
| 34 | " | " | " | " | 1 | H | " | 3 | -OH | HCl + 5,5% $H_2O$ | $C_{20}H_{27}ClN_2O_5$ + 5,5 % $H_2O$ | 434,80 | 120 | C.H.N. (+ 5,5 % $H_2O$) | | | |
| | | | | | | | | | | | | | | Cal. | 55,24 | 6,87 | 6,44 |
| | | | | | | | | | | | | | | Tr. | 55,31 | 6,91 | 6,65 |
| 35 | " | " | " | " | " | " | " | " | 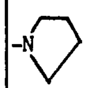 | HCl | $C_{24}H_{34}ClN_3O_4$ | 463,99 | 194 | C.H.N. | | | |
| | | | | | | | | | | | | | | Cal. | 62,12 | 7,39 | 9,06 |
| | | | | | | | | | | | | | | Tr. | 62,39 | 7,28 | 8,98 |
| 36 | " | " | " | " | 0 | " | " | 0 | $-NHCH_2COOH$ | Base | $C_{18}H_{21}N_3O_6$ | 375,37 | 220 | C.H.N. | | | |
| | | | | | | | | | | | | | | Cal. | 57,59 | 5,64 | 11,20 |
| | | | | | | | | | | | | | | Tr. | 57,40 | 5,81 | 10,92 |

TABLEAU I (suite)

| No. de Code | Ar- | R | $R_1$ | COA- | m | $R_2$ | $R_3$ | n | -B | Forme | Formule brute | Poids moléculaire | Point de fusion (°C) | ANALYSE ELEMENTAIRE OU $[\alpha]_D$ % | C | H | N |
|---|---|---|---|---|---|---|---|---|---|---|---|---|---|---|---|---|---|
| 37 | 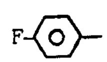 | -H | -H | 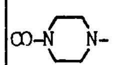 | 0 | $CH_3$ | H | 0 | -OH (±) | HCl | $C_{16}H_{20}ClFN_2O_3$ | 342,79 | 100 (décomposition) | C.H.N. | | | |
| | | | | | | | | | | | | | | Cal. | 56,06 | 5,88 | 8,17 |
| | | | | | | | | | | | | | | Tr. | 55,87 | 5,68 | 8,19 |
| 38 | 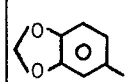 | " | " | " | " | H | " | " | $-NH-(CH_2)_2-N(CH_3)_2$ | di HCl + 0,9% $H_2O$ | $C_{20}H_{30}Cl_2N_4O_4$ + 0,9 % $H_2O$ | 465,43 | 222 (décomposition) | C.H.N. (+ 0,9 % $H_2O$) | | | |
| | | | | | | | | | | | | | | Cal. | 51,61 | 6,59 | 12,03 |
| | | | | | | | | | | | | | | Tr. | 51,56 | 6,53 | 11,90 |
| 39 | " | " | " | " | " | " | " | " | $-NH(CH_2)_2$ HOOC | HCl + 2,5%$H_2O$ | $C_{19}H_{24}ClN_3O_6$ + 2,5 % $H_2O$ | 436,78 | 120 | C.H.N. (+ 2,5 % $H_2O$) | | | |
| | | | | | | | | | | | | | | Cal. | 52,24 | 5,82 | 9,62 |
| | | | | | | | | | | | | | | Tr. | 52,33 | 6,02 | 9,62 |
| 40 | " | " | " | " | " | " | " | " | $-NH-(CH_2)_2$ $CONH_2$ | HCl | $C_{19}H_{25}ClN_4O_5$ | 424,88 | 180 | C.H.N. | | | |
| | | | | | | | | | | | | | | Cal. | 53,71 | 5,93 | 13,19 |
| | | | | | | | | | | | | | | Tr. | 53,46 | 6,09 | 12,88 |

TABLEAU I (suite)

| No. de Code | Ar- | R | $R_1$ | COA- | m | $R_2$ | $R_3$ | n | -B | Forme | Formule brute | Poids moléculaire | Point de fusion (°C) | ANALYSE ELEMENTAIRE OU $[\alpha]_D$ % | C | H | N |
|---|---|---|---|---|---|---|---|---|---|---|---|---|---|---|---|---|---|
| | | | | | | | | | | | | | | C.H.N. | | | |
| 41 | 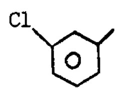 | H | H | 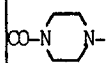 | 0 | $CH_3$ | H | 0 | -OH (±) | HBr | $C_{16}H_{20}BrClN_2O_3$ | 403,70 | 220 (décomposition) | Cal. | 47,60 | 4,99 | 6,94 |
| | | | | | | | | | | | | | | Tr. | 47,70 | 5,02 | 7,05 |
| | | | | | | | | | | | | | | C.H.N. (+ 5,4 % $H_2O$) | | | |
| 42 | 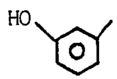 | " | " | " | " | " | " | " | " | Base + 5,4% $H_2O$ | $C_{16}H_{20}N_2O_4$ + 5,4 % $H_2O$ | 321,71 | 225 (décomposition) | Cal. | 59,73 | 6,86 | 8,71 |
| | | | | | | | | | | | | | | Tr. | 59,52 | 6,70 | 8,61 |
| | | | | | | | | | | | | | | C.H.N. (+ 2,6 % $H_2O$) | | | |
| 43 | 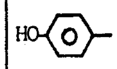 | " | " | " | " | " | " | " | " | Base + 2,6% $H_2O$ | $C_{16}H_{20}N_2O_4$ + 2,6 % $H_2O$ | 312,33 | > 250 | Cal. | 61,52 | 6,74 | 8,97 |
| | | | | | | | | | | | | | | Tr. | 61,61 | 6,79 | 8,89 |
| | | | | | | | | | | | | | | C.H.N. (1,3 oxalate+1,5% $H_2O$) | | | |
| 44 | 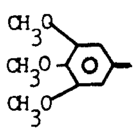 | " | " | 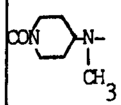 | " | H | " | " | 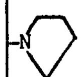 | 1,3 oxalate + 1,5% $H_2O$ | $C_{24}H_{35}N_3O_5$ + 1,3 oxalate + 1,5 % $H_2O$ | 571,16 | 105 | Cal. | 55,93 | 6,87 | 7,36 |
| | | | | | | | | | | | | | | Tr. | 55,66 | 7,07 | 7,34 |

TABLEAU I (suite)

| No. de Code | Ar- | R | $R_1$ | COA- | m | $R_2$ | $R_3$ | n | -B | Forme | Formule brute | Poids molécu-laire | Point de fusion (°C) | ANALYSE ELEMENTAIRE OU [α]$_D$ % | C | H | N |
|---|---|---|---|---|---|---|---|---|---|---|---|---|---|---|---|---|---|
| 45 | 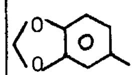 | H | H | 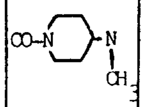 | O | H | H | O | 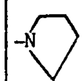 | Oxalate + 1 % $H_2O$ | $C_{24}H_{31}N_3O_8$ + 1 % $H_2O$ | 494,46 | 142 | C.H.N. (+ 1 % $H_2O$) | | | |
| | | | | | | | | | | | | | | Cal. | 58,29 | 6,43 | 8,49 |
| | | | | | | | | | | | | | | Tr. | 57,90 | 6,30 | 8,07 |
| 46 | 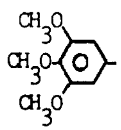 | " | " | 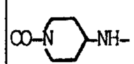 | " | $CH_3$ | " | " | 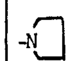 (±) | HCl + 1,6 % $H_2O$ | $C_{24}H_{36}ClN_3O_5$ + 1,6% $H_2O$ | 489,94 | 244 | C.H.N. (+ 1,6 % $H_2O$) | | | |
| | | | | | | | | | | | | | | Cal. | 58,83 | 7,59 | 8,58 |
| | | | | | | | | | | | | | | Tr. | 58,55 | 7,65 | 8,44 |
| 47 | 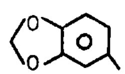 | " | " | " | " | " | " | " | " | HCl + 1,9% $H_2O$ | $C_{22}H_{30}ClN_3O_4$ + 1,9 % $H_2O$ | 444,38 | 240 | C.H.N. (+ 1,9 % $H_2O$) | | | |
| | | | | | | | | | | | | | | Cal. | 59,45 | 7,02 | 9,46 |
| | | | | | | | | | | | | | | Tr. | 59,49 | 7,10 | 9,26 |
| 48 | " | " | " | 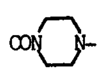 | " | " | " | " | $-NH-(CH_2)_2$ HOOC (±) | Base + 4,8% $H_2O$ | $C_{20}H_{25}N_3O_6$ + 4,8 % $H_2O$ | 423,59 | 100 | C.H.N. (+ 4,8 % $H_2O$) | | | |
| | | | | | | | | | | | | | | Cal. | 56,71 | 6,48 | 9,92 |
| | | | | | | | | | | | | | | Tr. | 56,72 | 6,59 | 10,08 |

TABLEAU I (suite)

| No. de Code | Ar- | R | $R_1$ | COA- | m | $R_2$ | $R_3$ | n | -B | Forme | Formule brute | Poids molécu-laire | Point de fusion (°C) | ANALYSE ELEMENTAIRE OU $[\alpha]_D$ % | C | H | N |
|---|---|---|---|---|---|---|---|---|---|---|---|---|---|---|---|---|---|
| 49 | 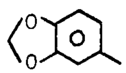 | H | H | 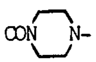 | 0 | $CH_3$ | H | 0 | $-NH-(CH_2)_2$ $H_2NOC$ (±) | Base | $C_{20}H_{26}N_4O_5$ | 402,44 | 183 | C.H.N. | | | |
| | | | | | | | | | | | | | | Cal. | 59,69 | 6,51 | 13,92 |
| | | | | | | | | | | | | | | Tr. | 59,57 | 6,69 | 13,89 |
| 50 | " | " | " | " | " | H | " | " | $-NH-(CH_2)_2-N$ Et Et | di HCl + 1,05%$H_2O$ | $C_{22}H_{34}Cl_2N_4O_4$ + 1,05 % $H_2O$ | 494,63 | 220 | C.H.N. (+ 1,05 % $H_2O$) | | | |
| | | | | | | | | | | | | | | Cal. | 53,41 | 7,05 | 11,33 |
| | | | | | | | | | | | | | | Tr. | 53,42 | 7,10 | 11,05 |
| 51 | " | " | " | " | " | $C_3H_7$ n | H | 0 | 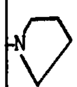 (±) | Maléate | $C_{27}H_{35}N_3O_8$ | 529,57 | 200 | C.H.N. | | | |
| | | | | | | | | | | | | | | Cal. | 61,23 | 6,66 | 7,94 |
| | | | | | | | | | | | | | | Tr. | 61,09 | 6,76 | 7,98 |
| 52 | " | " | " | " | " | $CH_3$ | $CH_3$ | " | 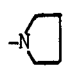 | Base + 0,9 %$H_2O$ | $C_{22}H_{29}N_3O_4$ + 0,9 % $H_2O$ | 403,02 | 165 | C.H.N. (+ 0,9 % $H_2O$) | | | |
| | | | | | | | | | | | | | | Cal. | 65,56 | 7,36 | 10,43 |
| | | | | | | | | | | | | | | Tr. | 65,70 | 7,56 | 10,21 |

TABLEAU I (suite)

| No. de Code | Ar- | R | $R_1$ | COA- | m | $R_2$ | $R_3$ | n | -B | Forme | Formule brute | Poids moléculaire | Point de fusion (°C) | ANALYSE ELEMENTAIRE OU $[\alpha]_D$ % | C | H | N |
|---|---|---|---|---|---|---|---|---|---|---|---|---|---|---|---|---|---|
| 53 | 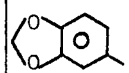 | H | H | 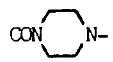 | O | $C_3H_7$ iso | H | O | 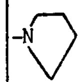 (±) | Base | $C_{22}H_{31}N_3O_4$ | 413,50 | 100 | C.H.N. | | | |
| | | | | | | | | | | | | | | Cal. | 66,80 | 7,56 | 10,16 |
| | | | | | | | | | | | | | | Tr. | 66,52 | 7,69 | 9,87 |
| 54 | " | " | " | " | " | H | " | " | 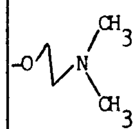 | 1,5 Maléate | $C_{26}H_{33}N_3O_{11}$ | 563,55 | 154 | C.H.N. | | | |
| | | | | | | | | | | | | | | Cal. | 55,41 | 5,90 | 7,46 |
| | | | | | | | | | | | | | | Tr. | 55,13 | 5,93 | 7,36 |
| 55 | " | " | " | " | " | " | " | " | 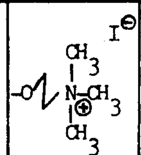 | - | $C_{21}H_{30}IN_3O_5$ | 531,38 | 222 | C.H.N. | | | |
| | | | | | | | | | | | | | | Cal. | 47,46 | 5,69 | 7,91 |
| | | | | | | | | | | | | | | Tr. | 47,32 | 5,82 | 7,91 |
| 56 | " | " | " | 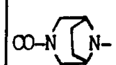 | " | " | " | " | 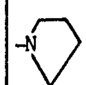 | Base + 4 % $H_2O$ | $C_{22}H_{27}N_3O_4$ + 4 % $H_2O$ | 413,36 | Produit vitreux | C.H.N. (+ 4 % $H_2O$) | | | |
| | | | | | | | | | | | | | | Cal. | 63,82 | 7,03 | 10,15 |
| | | | | | | | | | | | | | | Tr. | 63,63 | 7,18 | 10,26 |

TABLEAU I (suite)

| No. de Code | Ar- | R | $R_1$ | COA- | m | $R_2$ | $R_3$ | n | -B | Forme | Formule brute | Poids moléculaire | Point de fusion (°C) | ANALYSE ELEMENTAIRE OU $[\alpha]_D$ % | C | H | N |
|---|---|---|---|---|---|---|---|---|---|---|---|---|---|---|---|---|---|
| 57 | 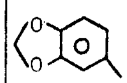 | H | H | 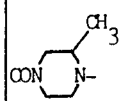 | O | H | H | O | 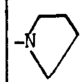 | HCl | $C_{21}H_{28}ClN_3O_4$ | 421,91 | 220 (décomposition) | C.H.N. | | | |
| | | | | | | | | | | | | | | Cal. | 59,78 | 6,69 | 9,96 |
| | | | | | | | | | | | | | | Tr. | 59,60 | 6,84 | 10,15 |
| 58 | 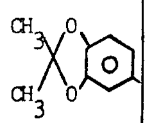 | " | " | 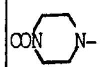 | " | " | " | " | " | 1,5 oxalate + 0,75% $H_2O$ | $C_{25}H_{32}N_3O_{10}$ + 0,75 % $H_2O$ | 538,57 | 200 | C.H.N. (+ 0,75 % $H_2O$) | | | |
| | | | | | | | | | | | | | | Cal. | 55,74 | 6,06 | 7,80 |
| | | | | | | | | | | | | | | Tr. | 55,90 | 6,11 | 7,82 |
| 59 | 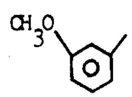 | " | " | " | " | $CH_3$ | " | " | -OH | HBr | $C_{17}H_{23}BrN_2O_4$ | 399,28 | 220 (décomposition) | C.H.N. | | | |
| | | | | | | | | | | | | | | Cal. | 51,13 | 5,81 | 7,02 |
| | | | | | | | | | | | | | | Tr. | 50,96 | 5,96 | 7,07 |
| 60 | 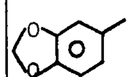 | " | " | 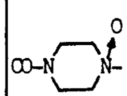 | " | H | " | " | 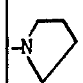 | + 3% $H_2O$ | $C_{20}H_{25}N_3O_5$ + 3 % $H_2O$ | 399,41 | 150 (décomposition) | C.H.N. (+ 3% $H_2O$) | | | |
| | | | | | | | | | | | | | | Cal. | 60,14 | 6,63 | 10,52 |
| | | | | | | | | | | | | | | Tr. | 59,99 | 6,68 | 10,60 |

TABLEAU I (suite)

| No. de Code | Ar- | R | $R_1$ | COA- | m | $R_2$ | $R_3$ | n | -B | Forme | Formule brute | Poids moléculaire | Point de fusion (°C) | ANALYSE ELEMENTAIRE OU $[\alpha]_D$ % | C | H | N |
|---|---|---|---|---|---|---|---|---|---|---|---|---|---|---|---|---|---|
| | | | | | | | | | | | | | | C.H.N. (+ 6,5 % $H_2O$) | | | |
| 61 | 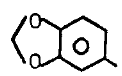 | H | H | 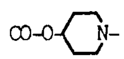 | O | H | H | O | 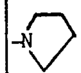 | HCl + 6,5 %$H_2O$ | $C_{21}H_{27}ClN_2O_5$ + 6,5 % $H_2O$ | 452,30 | 175 puis 250 | Cal. | 55,76 | 6,75 | 6,19 |
| | | | | | | | | | | | | | | Tr. | 55,91 | 6,69 | 6,09 |
| | | | | | | | | | | | | | | C.H.N. | | | |
| 62 | " | " | " | 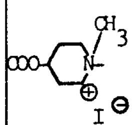 | " | " | " | " | " | - | $C_{22}H_{29}N_2O_5I$ | 528,37 | 212 | Cal. | 50,01 | 5,53 | 5,30 |
| | | | | | | | | | | | | | | Tr. | 49,84 | 5,51 | 5,37 |
| | | | | | | | | | | | | | | C.H.N. (+ 1 % $H_2O$) | | | |
| 63 | " | " | " | 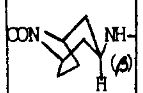 | " | " | " | " | " | HCl + 1 %$H_2O$ | $C_{23}H_{29}N_3O_4$ + 1 % $H_2O$ | 452,48 | > 260 | Cal. | 61,05 | 6,79 | 9,29 |
| | | | | | | | | | | | | | | Tr. | 60,24 | 6,80 | 9,11 |
| | | | | | | | | | | | | | | C.H.N. | | | |
| 64 | 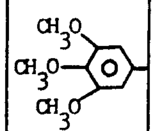 | " | " | 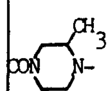 | " | " | " | " | " | Base | $C_{23}H_{33}N_3O_5$ | 431,52 | 100 (décomposition) | Cal. | 64,01 | 7,71 | 9,74 |
| | | | | | | | | | | | | | | Tr. | 63,69 | 7,80 | 9,69 |

TABLEAU I (suite)

| No. de Code | Ar- | R | $R_1$ | COA- | m | $R_2$ | $R_3$ | n | -B | Forme | Formule brute | Poids moléculaire | Point de fusion (°C) | ANALYSE ELEMENTAIRE OU $[\alpha]_D$ % | C | H | N |
|---|---|---|---|---|---|---|---|---|---|---|---|---|---|---|---|---|---|
| 65 | 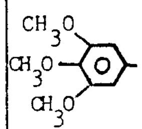 | H | H | 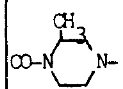 | O | H | H | O | 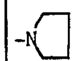 | Base + 1,8 % $H_2O$ | $C_{23}H_{33}N_3O_5$ + 1,8 % $H_2O$ | 439,43 | 50 (décomposition) | C.H.N. (+1,8 % $H_2O$) | | | |
| | | | | | | | | | | | | | | Cal. | 62,86 | 7,77 | 9,56 |
| | | | | | | | | | | | | | | Tr. | 63,00 | 7,87 | 9,61 |
| 66 | 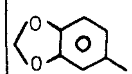 | " | " | " | " | " | " | " | " | HCl + 3 % $H_2O$ | $C_{21}H_{28}ClN_3O_4$ + 3 % $H_2O$ | 434,96 | 50 (décomposition) | C.H.N. (HCl + 3 % $H_2O$) | | | |
| | | | | | | | | | | | | | | Cal. | 58,17 | 6,83 | 9,66 |
| | | | | | | | | | | | | | | Tr. | 57,65 | 7,38 | 9,93 |
| 67 | 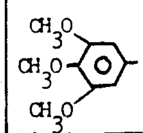 | " | " | 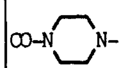 | " | $CH_3$ | " | " | -OH (±) | Base + 3,75 % $H_2O$ | $C_{19}H_{26}N_2O_6$ + 3,75 % $H_2O$ | 393,16 | 202 | C.H.N. (+ 3,75 % $H_2O$) | | | |
| | | | | | | | | | | | | | | Cal. | 58,04 | 7,09 | 7,12 |
| | | | | | | | | | | | | | | Tr. | 58,12 | 6,58 | 7,03 |
| 68 | " | " | " | 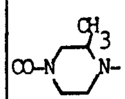 | " | " | " | " | 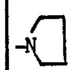 | Base + 1,63 % $H_2O$ | $C_{24}H_{35}N_3O_5$ + 1,63 % $H_2O$ | 452,93 | 50 (décomposition) | C.H.N. (+ 1,63 % $H_2O$) | | | |
| | | | | | | | | | | | | | | Cal. | 63,64 | 7,97 | 9,28 |
| | | | | | | | | | | | | | | Tr. | 63,83 | 8,11 | 9,35 |

TABLEAU I (suite)

| No. de Code | Ar- | R | R1 | COA- | m | R2 | R3 | n | -B | Forme | Formule brute | Poids moléculaire | Point de fusion (°C) | ANALYSE ELEMENTAIRE OU [α]D % | C | H | N |
|---|---|---|---|---|---|---|---|---|---|---|---|---|---|---|---|---|---|
| 69 | 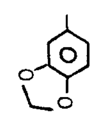 | H | H | 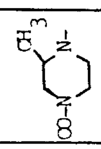 | 0 | $OCH_3$ | H | 0 | 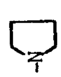 | Base + 2,6 % $H_2O$ | $C_{22}H_{29}N_3O_4$ + 2,6 % $H_2O$ | 410,10 | 50 (décomposition) | C.H.N. (+ 2,6 % $H_2O$) | | | |
| | | | | | | | | | | | | | | Cal. | 64,43 | 7,42 | 10,25 |
| | | | | | | | | | | | | | | Tr. | 64,38 | 7,51 | 10,15 |

Les composés selon l'invention ont été étudiés chez l'animal de laboratoire et ont montré des activités pharmacologiques et notamment des activités stimulantes, protectrices et/ou correctrices des fonctions cérébrales.

Ces activités ont été mises en évidence notamment par le test de la rétention mnésique de l'activité exploratoire effectué selon le protocole suivant :

On mesure dans un appareil ACTIMETRE APELAB [BOISSIER et SIMON, Arch. Inter. Pharmacodyn. 158, 212, (1965)], l'activité exploratoire pendant 5 minutes de souris males SWISS-WEBSTER, puis les animaux reçoivent une injection par voie intrapéritonéale (ou par voieorale), des composés selon l'invention ou de sérum physiologique. Après une semaine, on mesure de nouveau l'activité exploratoire des animaux traités et l'effet sur la rétention mnésique est mesurée par une habituation, c'est-à-dire par une diminution statistiquement significative (t de STUDENT par groupes appariés) de l'activité exploratoire. Pour illustrer l'invention, nous donnons dans le tableau II ci-dessous, les résultats obtenus avec quelques composés selon l'invention. La toxicité aiguë approchée est déterminée selon la méthode décrite par MILLER et TAINTER dans Proc. Soc. Exp. Biol. Med. 57, 261 (1944). Les résultats obtenus avec quelques composés selon l'invention sont également rassemblés, à titre d'exemples, dans ce tableau II.

TABLEAU II

| Composés testés | Test de la rétention mnésique | | Toxicité aiguë (souris) | |
|---|---|---|---|---|
| Numéro de code | Dose (souris) mg/kg/i.p. | % de diminution de l'activité exploratoire | Dose mg/kg/i.p. | Mortalité % |
| 1 | 10 | 26,3 | - | - |
| 2 | 1 | 21,8 | 400 | 0 % |
| 6 | 0,01 | 22,7 | 400 | 0 % |
| 9 | 0,1 | 30,5 | " | " |
| 17 | 0,01 | 23,9 | " | " |
| 18 | 0,01 | 21,6 | " | " |
| 22 | 0,01 | 21,3 | " | " |
| 23 | 0,01 | 33,5 | " | " |
| 31 | 0,01 | 26,5 | " | " |
| 34 | 0,001 | 24,9 | " | " |
| 47 | 0,1 | 26,9 | " | " |

Comme le montrent les résultats précédents, les composés selon l'invention présentent une activité pharmacologique marquée et une faible toxicité. Les composés selon l'invention pharmaceutiquement acceptables trouvent donc leur application enthérapeutique comme médicaments utiles notamment pour stimuler l'efficience intellectuelle chez le sujet normal, pour préserver les fonctions cérébrales chez le sujet âgé et pour traiter les troubles de la vigilance et/ou de la mémorisation, consécutifs à diverses pathologies, notamment les traumatismes crâniens, les commotions cérébrales ou les accidents cérébrovasculaires aigus ou subaigus.

La présente invention s'étend par ailleurs aux compositions pharmaceutiques renfermant comme principe actif, l'un au moins des médicaments définis ci-dessus, ces compositions pouvant être formulées notamment en vue de leur administration orale ou parentérale. Ainsi, elles pourront par exemple être administrées par voie orale sous forme de dragées, gélules, comprimés ou sous forme de soluté buvable, à des doses pouvant aller jusqu'à 2,5 g de principe actif/jour en plusieurs prises par jour (jusqu'à six prises) ou par voie parentérale sous forme d'ampoules injectables contenant jusqu'à 1 g de principe actif (1 à 3 injections journalières).

Dans le cas de l'administration orale sous forme de dragées, gélules et comprimés, ces derniers pourront avantageusementcontenir un véhicule (tel que dérivés cellulosiques, polymères vinyliques ou gommes) qui permet une modulation de la libération du principe actif. Les solutés buvables seront avantageusement des solutions ou suspensions aqueuses (véhicule = eau ) ou des solutions ou suspensions partiellement aqueuses (véhicule = eau + alcool, eau + glycérine ou eau + propylène glycol). Enfin, dans le cas de l'administration par voie parentérale, le principe actif pourra être injecté sous la forme de suspensions ou de solutions injectables de lyophilisats contenant ce principe actif.

## Revendications

**1.** Dérivé cinnamoïque de formule :

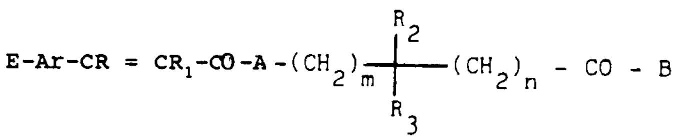 (I)

dans laquelle l'ensemble (Ar, R, $R_1$,COA, m, $R_2$, $R_3$, n, B) prend l'une des significations suivantes :

-( 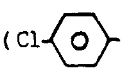 , H, H, 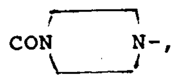 , O, $CH_3$, H, O, OH) (±),

-( 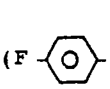 , H, H, 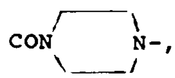 , O, $CH_3$, H, O, OH) (±),

-( 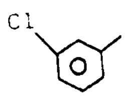 , H, H, 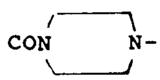 , O, $CH_3$, H, O, OH) (±),

-( 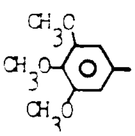 , H, H, 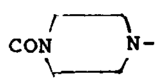 , O, $CH_3$, H, O, OH) (±),

-( 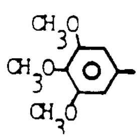 , H, H, 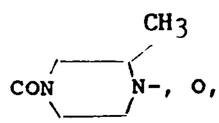 , O, H, H, O, 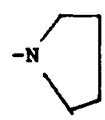 ),

-( 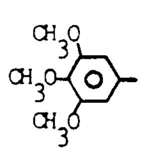 , H, H, 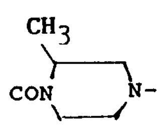 , O, H, H, O, 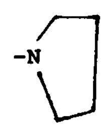 ),

-( 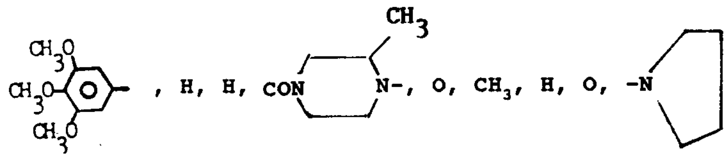 , H, H, , O, $CH_3$, H, O, ),

-( 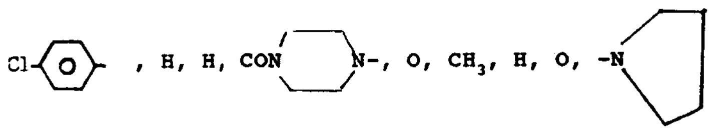 , H, H, , O, $CH_3$, H, O, ) (±),

-( 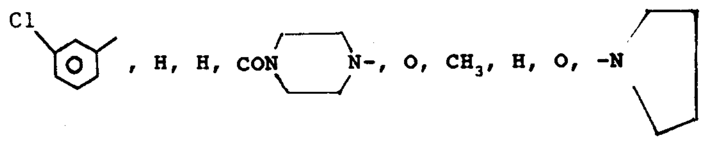 , H, H, , O, $CH_3$, H, O, ) (±),

-( 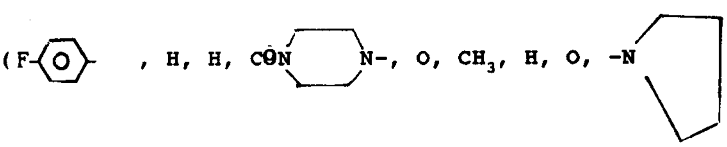 , H, H, , O, $CH_3$, H, O, ) (±),

-( 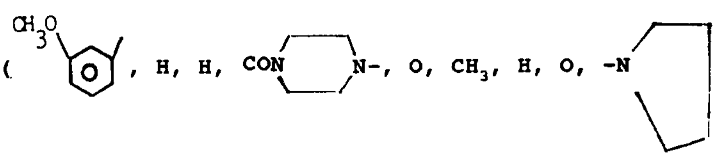 , H, H, , O, $CH_3$, H, O, ) (±),

-( 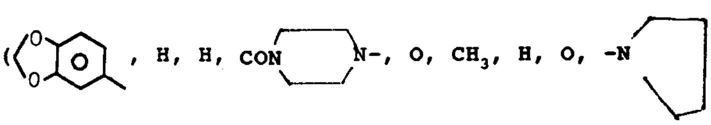 , H, H, , O, $CH_3$, H, O, ) (±),

-( 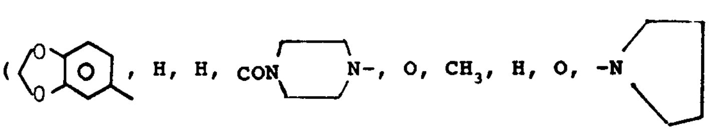 , H, H, , O, $CH_3$, H, O, ) (S+),

-( 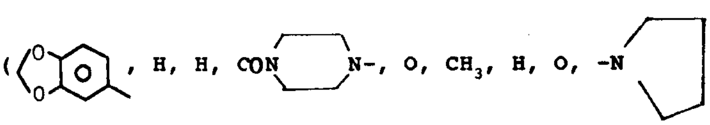 , H, H, , O, $CH_3$, H, O, ) (R-),

-( 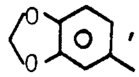, H, H, 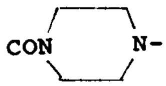, 1, H, H, 2, 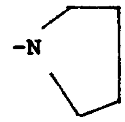 ),

-( 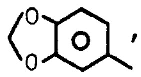, H, H, 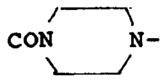, O, H, H, O, 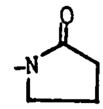 ),

-( 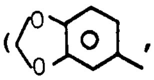, H, H, 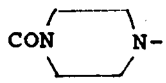, O, H, H, O, $-NH(CH_2)_3-COOH$),

-( 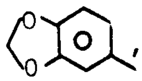, H, H, 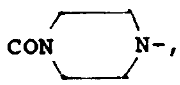, O, H, H, O, $-NHCH_2-COOH$),

-( 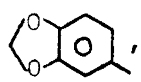, H, H, 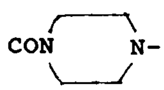, O, H, H, O, $-NH(CH_2)_2-COOH$),

-( 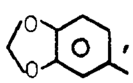, H, H, 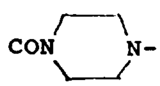, O, $CH_3$, H, O, $-NH(CH_2)_2-COOH$) (±),

-( 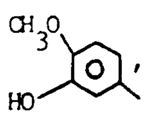, H, H, 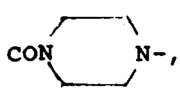, O, H, H, O, $-NH(CH_2)_3-COOH$),

-( 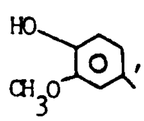, H, H, 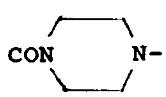, O, H, H, O, $-NH(CH_2)_3-COOH$),

-( 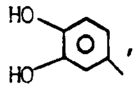, H, H, CON 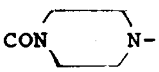 N-, O, H, H, O, $-NH(CH_2)_3-COOH$),

-( 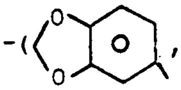, H, H, CON 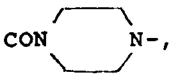 N-, O, $CH_3$, H, O, $-NHCH_2CONH_2$),

-( 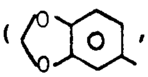, H, H, CON 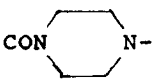 N-, O, $CH_3$, H, O, $-NH(CH_2)_2-CONH_2$) (±),

-( 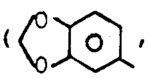, H, H, CON 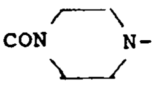 N-, O, H, H, O, $-NH(CH_2)_2-CONH_2$),

-( 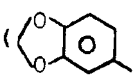, H, H, CON 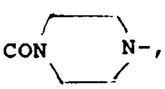 N-, O, H, H, O, $-O(CH_2)_2N(CH_3)_2$),

-( 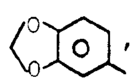, H, H, CON 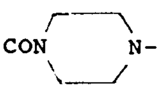 N-, O, H, H, O, $-NH(CH_2)_2N(CH_3)_2$),

-( 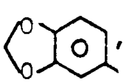, H, H, CON 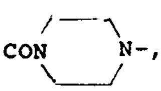 N-, O, H, H, O, $-NH(CH_2)_2N(Et)_2$),

ainsi que ses sels d'addition d'acides ou de bases organiques ou minéraux, son N-oxyde, son ammonium quaternaire et ses hydrates.

**2.** Composition pharmaceutique, caractérisée en ce qu'elle comprend à titre de principe actif au moins un composé selon la revendication 1, en association avec un véhicule pharmaceutiquement acceptable.

**3.** Utilisation des composés objet de la revendication 1 pour la fabrication de médicaments à activités stimulantes, protectrice et/ou correctrices des fonctions cérébrales.

## Claims

**1.** Cinnamoic derivative of formula :

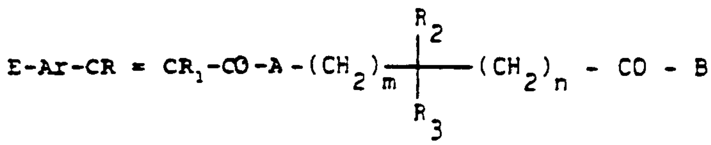 (I)

wherein the set (Ar, R, $R_1$,COA, m, $R_2$ $R_3$, n, B) has any one of the following meanings :

-( 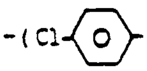 , H, H, 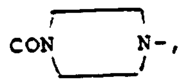 , O, $CH_3$, H, O, OH) (±),

-( 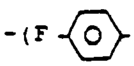 , H, H, 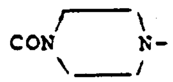 , O, $CH_3$, H, O, OH) (±),

-( 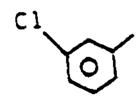 , H, H, 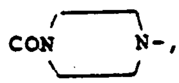 , O, $CH_3$, H, O, OH) (±),

-( 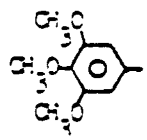 , H, H, 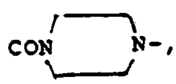 , O, $CH_3$, H, O, OH) (±),

-( 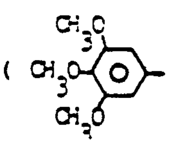 , H, H, 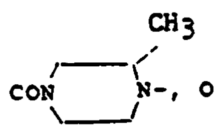 , O, H, H, O, 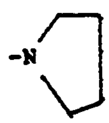 ),

-( 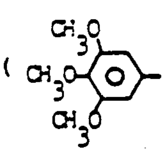 , H, H, 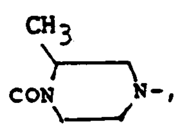 , O, H, H, O, 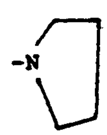 ),

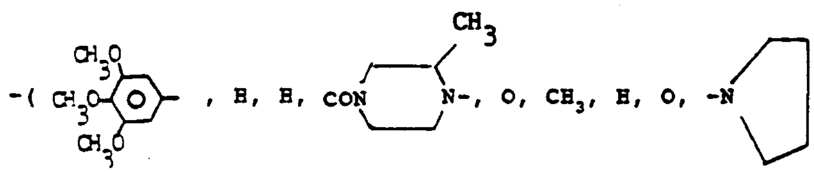
),

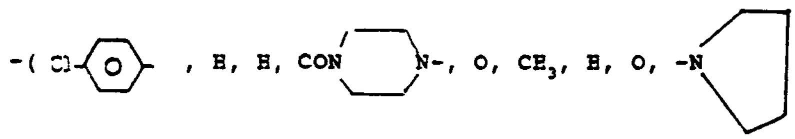
) (±),

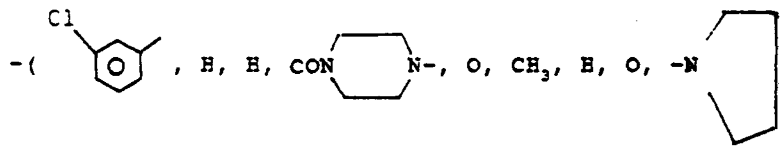
) (±),

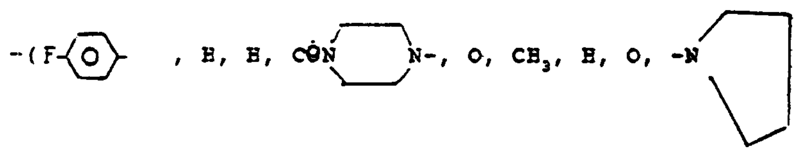
) (±),

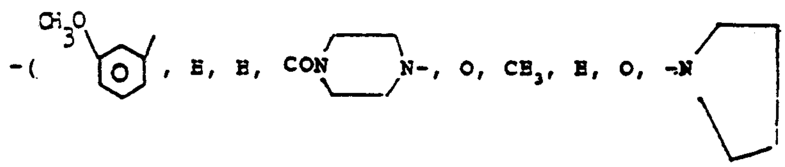
) (±),

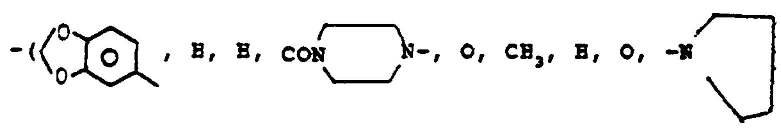
) (±),

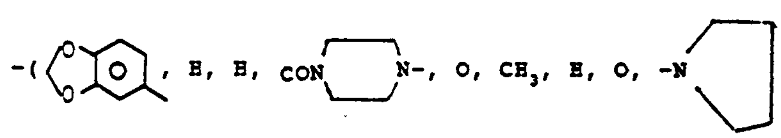
) (S+),

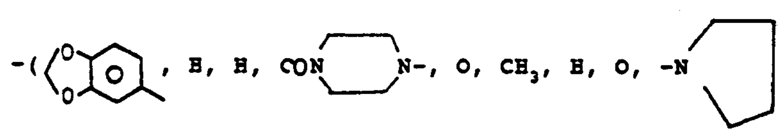
) (R-),

-( 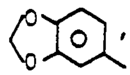, H, H, 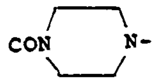, 1, H, H, 2, 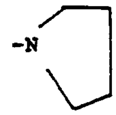 ),

-( 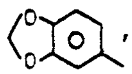, H, H, 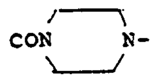, O, H, H, O, 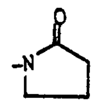 ),

-( 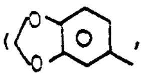, H, H, 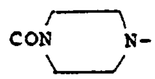, O, H, H, O, $-NH(CH_2)_3-COOH$),

-( 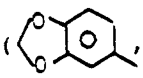, H, H, 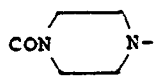, O, H, H, O, $-NHCH_2-COOH$),

-( 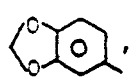, H, H, 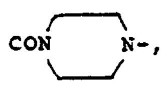, O, H, H, O, $-NH(CH_2)_2-COOH$),

-( 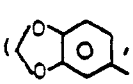, H, H, 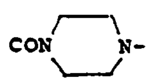, O, $CH_3$, H, O, $-NH(CH_2)_2-COOH$) (±),

-( 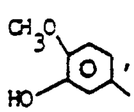, H, H, 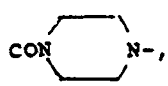, O, H, H, O, $-NH(CH_2)_3-COOH$),

-( 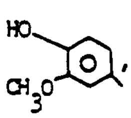, H, H, 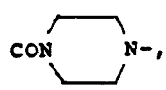, O, H, H, O, $-NH(CH_2)_3-COOH$),

-( 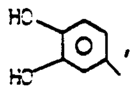, H, H, 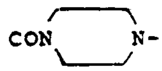 O, H, H, O, $-NH(CH_2)_3-COOH$),

-( 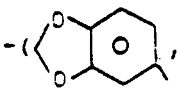, H, H, 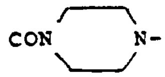 O, $CH_3$, H, O, $-NHCH_2CONH_2$),

-( 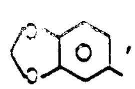, H, H, 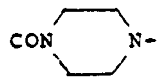 O, $CH_3$, H, O, $-NH(CH_2)_2-CONH_2$) (±),

-( 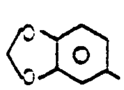, H, H, 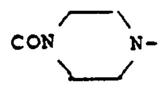 O, H, H, O, $-NH(CH_2)_2-CONH_2$),

-( 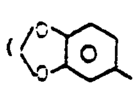, H, H, 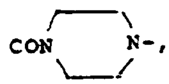 O, H, H, O, $-O(CH_2)_2N(CH_3)_2$),

-( 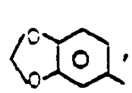, H, H, 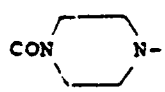 O, H, H, O, $-NH(CH_2)_2N(CH_3)_2$),

-( 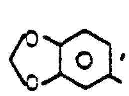, H, H. 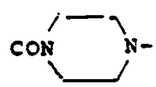 O, H, H, O, $-NH(CH_2)_2N(Et)_2$),

as well as its organic or mineral acid or base addition salts, N-oxide, quaternary ammonium and hydrates.

**2.** Pharmaceutical composition, characterized in that it comprises as active principle at least one compound according to claim 1, in combination with a pharmaceutically acceptable excipient.

**3.** Use of the compounds according to claim 1 for the manufacture of drugs having stimulating, protecting and / or correcting activities of the cerebral functions.

**Patentansprüche**

**1.** Cinnamoylderivat der Formel

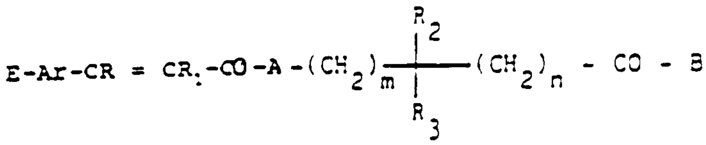 (I)

worin die Gesamtheit (Ar, R, $R_1$, COA, m, $R_2$, $R_3$, n, B) eine der folgenden Bedeutungen hat:

-( 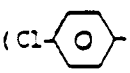 , H, H, 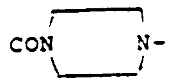 , O, $CH_3$, H, O, OH) (±),

-( 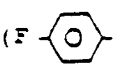 , H, H, 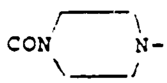 , O, $CH_3$, H, O, OH) (±),

-( 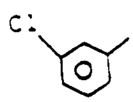 , H, H, 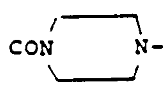 , O, $CH_3$, H, O, OH) (±),

-( 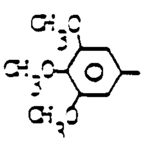 , H, H, 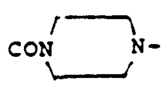 , O, $CH_3$, H, O, OH) (±),

-( 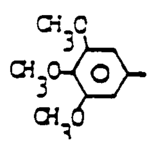 , H, H, 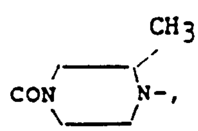 , O, H, H, O, 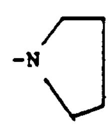 ),

-( 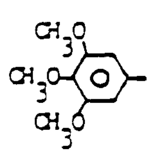 , H, H, 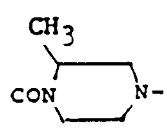 , O, H, H, O, 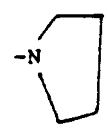 ),

-( 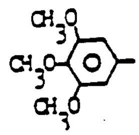 , H, H, 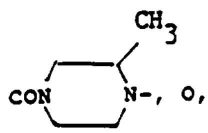 , O, $CH_3$, H, O, 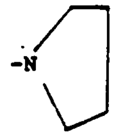 ),

-( 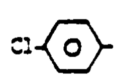 , H, H, 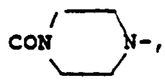 , O, $CH_3$, H, O, 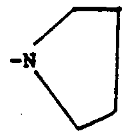 ) (±),

-( 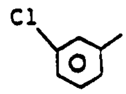 , H, H, 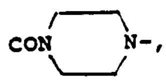 , O, $CH_3$, H, O, 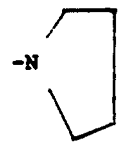 ) (±),

-( 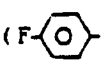 , H, H, 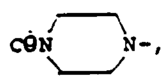 , O, $CH_3$, H, O, 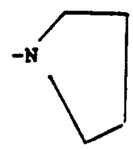 ) (±),

-( 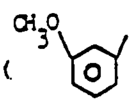 , H, H, 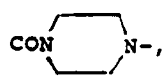 , O, $CH_3$, H, O, 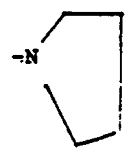 ) (±),

-( 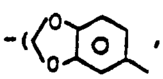 , H, H, 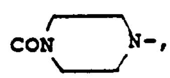 , O, $CH_3$, H, O, 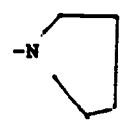 ) (±),

-( 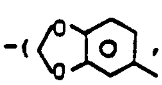 , H, H, 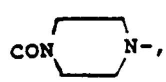 , O, $CH_3$, H, O, 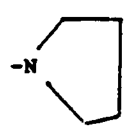 ) (S+),

-( 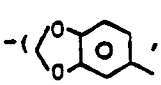 , H, H, 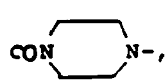 , O, $CH_3$, H, O, 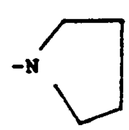 ) (R-),

-( 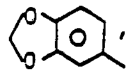 , H, H, 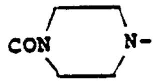 , 1, H, H, 2, 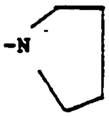 ),

-( 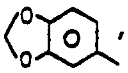 , H, H, 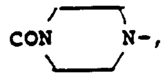 , 0, H, H, 0, 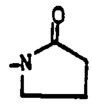 ),

-( 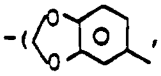 , H, H, 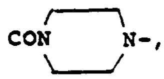 , 0, H, H, 0, $-NH(CH_2)_3-COOH$),

-( 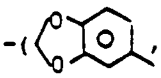 , H, H, 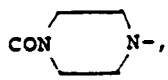 , 0, H, H, 0, $-NHCH_2-COOH$),

-( 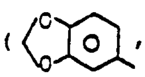 , H, H, 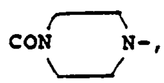 , 0, H, H, 0, $-NH(CH_2)_2-COOH$),

-( 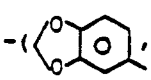 , H, H, 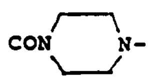 , 0, $CH_3$, H, 0, $-NH(CH_2)_2-COOH$) (±),

-( 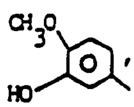 , H, H, 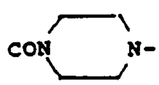 , 0, H, H, 0, $-NH(CH_2)_3-COOH$),

-( 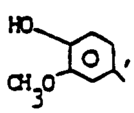 , H, H, 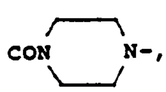 , 0, H, H, 0, $-NH(CH_2)_3-COOH$),

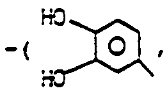, H, H, 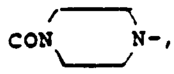, O, H, H, O, $-NH(CH_2)_3-COOH)$,

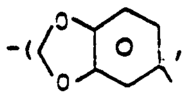, H, H, 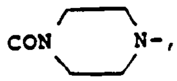, O, $CH_3$, H, O, $-NHCH_2CONH_2)$,

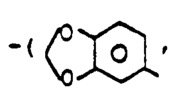, H, H, 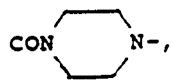, O, $CH_3$, H, O, $-NH(CH_2)_2-CONH_2)$ (±),

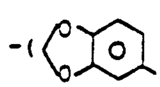, H, H, 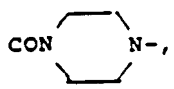, O, H, H, O, $-NH(CH_2)_2-CONH_2)$,

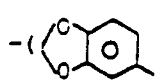, H, H, 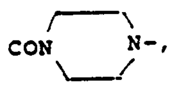, O, H, H, O, $-O(CH_2)_2N(CH_3)_2)$,

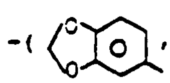, H, H, 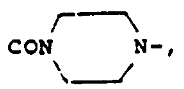, O, H, H, O, $-NH(CH_2)_2N(CH_3)_2)$,

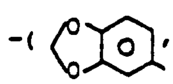, H, H. 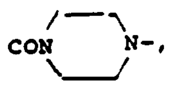, O, H, H, O, $-NH(CH_2)_2N(Et)_2)$,

ebenso wie seine Additionssalze von organischen oder anorganischen Basen oder Säuren, sein N-Oxid, sein quartäres Ammonium und seine Hydrate.

2. Pharmazeutische Zusammensetzung, dadurch gekennzeichnet, daß sie als aktives Prinzip wenigstens eine Verbindung nach Anspruch 1, zusammen mit einem pharmazeutisch annehmbaren Träger, umfaßt.

3. Verwendung der Verbindungen nach Anspruch 1 zur Herstellung von Medikamenten mit die Gehirnfunktion stimulierenden, schützenden und/oder korrigierenden Aktivitäten.